# EUROPEAN PATENT APPLICATION

(11) **EP 1 132 483 A2**
(43) Date of publication of application: **12.09.2001**
(21) Application number: 01105259.4
(22) Date of filing: 05.03.2001
(51) Int. Cl.: C12Q 1/68, G01N 33/53

(54) **Method for diagnosing schizophrenia using objective indices**

(30) Priority: 07.03.2000 JP 2000061775
(71) Applicant: President of Niigata University, Niigata City 950-2181 (JP)
(72) Inventor: Nawa, Hiroyuki, Niigata-shi, Niigata-ken (JP); Takahashi, Hitoshi, Niigata-shi, Niigata-ken (JP); Iritani, Shuji, Tokyo (JP)
(74) Representative: Draudt, Jutta, Dr.

(57) **Abstract**

Provided is a method for diagnosing whether or not a subject suffers from schizophrenia, comprising the steps of taking a sample containing nucleic acid and/or protein from the subject, quantifying nucleic acid and/or protein corresponding to at least one gene selected from the group consisting of genes listed in Table 1 below, fragments thereof, and complementary nucleic acid thereof, and diagnosing whether or not the subject suffers from schizophrenia by using a quantitative value of at least one protein, a fragment thereof or the nucleic acid.

## Description

The present invention relates to a method of objectively diagnosing schizophrenia by using an expression amount of nucleic acid encoding defined protein (index gene) as an index.

Schizophrenia is a mental disorder. About 0.8% of the population suffers from schizophrenia during their youth. Once people suffer from schizophrenia, it takes a long time to recover from it. Public loss caused by schizophrenia comes to be immeasurably large.

To develop therapies and diagnoses for schizophrenia, aggressive studies have been made in many institutes all over the world. In particular, significant progress has been made on therapies since dopamine receptor antagonistic drugs such as chlorpromazine were developed.

In contrast, schizophrenia is still diagnosed on the basis of psychological symptoms and classified into a paranoid type, catatonic type, disorganized type, and difficult to diagnose type, even in the latest diagnostic reference "DSMIV". Schizophrenia is, therefore, finally diagnosed subjectively by physicians depending upon their personal judge. Hence, it may not be said that diagnosis is made accurately.

Under these circumstances, chromosomal mapping of a causal gene developing schizophrenia and identification of the causal gene have been made aggressively. However, definitive reports have been not yet made.

The present invention was made to solve the aforementioned problems. An object of the present invention is to provide a method of objectively diagnosing schizophrenia by use of gene expression as an index.

According to the present invention, there is provided a method of diagnosing whether or not said subject suffers from schizophrenia, comprising the steps of:
taking a sample containing, for example, nucleic acid and/or protein from the subject;
quantifying nucleic acid and/or protein corresponding to at least one gene selected from the group consisting of genes listed below, fragments thereof, and complementary nucleic acid thereof:
   CCAAT-binding transcription factor subunit B (M59079);
   Transcription regulating interferon stimulating gene factor 3 γ subunit (M87503);
   DNA topoisomerase 1 (JO3250);
   Migration inhibitory factor related protein 8 (X06234);
   Growth arrest & DNA-damage inducible protein (M60974);
   MacMARCKS(X70326);
   ERBB-3 receptor protein-tyrosine kinase precursor (M29366, M34309);
   Proto-oncogene c-jun (JO4111);
   Phospholipase A2 (M86400);
   Erythrocyte urea transporter (U35735);
   T-lymphocyte maturation-associated protein MAL(M15800);
   Calcium/calmodulin-dependent protein kinase type IV catalytic subunit(L24959);
   Interleukin-10 precursor (M57627);
   Vascular endothelial growth factor precursor (M32977, M27281);
   Defender against cell death 1 (D15057);
   Zinc-finger DNA-binding protein (D45132);
   Bc12 homologous antagonist/killer (U23765; U16811; X84213);
   3 '5'-CAMP phosphodiesterase HPDE4A6 (U18087);
   Xeroderma pigmentosum group D complementing protein (X52221);
   Endothelin receptor type A (L06622),
   Epithelial discoidin domain receptor 1 precursor(X74979);
   Tyk2 non-receptor protein tyrosine kinase (X54637);
   Ets-associated protein tel (U11732);
   Platelet-derived growth factor A subunit precursor (X06374);
   FAN protein (X96586);
   Protein-tyrosine phosphatase γ precursor (L09247);
   EB1 protein (U24166);
   Ras related protein RAP-1A (M22995);
   Myelin-associated oligodendrocytic basic protein (D28113);
   Myelin basic protein (M13577);
   Brain-derived neurotrophic factor receptor (U12140);
   Gamma-aminobutyric acid (GABA) receptor β-1 subunit precursor (X14767);
   23k-Da highly basic protein (X56932);
   phosphatidylinositol-4-phosphate-5-kinase type III (S78798+U14957);
   Recoverin (S43855),
   HLA class histocompatibility antigen C-4 α subunit(M11886);
   P21-activated kinase α (U24152);
   Brain-specific tubulin α1 subunit (K00558);
   Ras related protein RAB-11B (X79780);
   Bone morphogenetic protein 3 (M22491);
   Apoptosis regulator bcl 2 (M14745);
   Xenoderma pigmentosum group B complementing protein (M31899);
   Acidic fibroblast growth factor (X65778+X51943+M13361);
   Neural cell adhesion molecule phosphatidylinositol-linked isoform precursor (X16841; S71824);
   Bc12 and p53 linked protein Bbp (U58334);
   Induced myeloid leukemia cell differentiation protein MCL-1(L08246);
   CD59 glycoprotein precursor (M334671);
   Neurotrophin-4 (M86528+S41522+S41540+S41541); and
   diagnosing whether or not the subject suffers from schizophrenia by using a quantitative value of said at least one nucleic acid.

This summary of the invention does not necessarily describe all necessary features so that the invention may also be a sub-combination of these described features.

The invention can be more fully understood from the following detailed description when taken in conjunction with the accompanying drawings, in which:
FIG. 1 is a view showing the results of Example 1; and
FIG. 2 is a view showing the results of Example 2.

The present invention is made based on the finding of the present inventors that the expression amounts of 52 kinds of genes listed in Table 1 below vary with statistical significance in schizophrenic patients. The present inventors successfully identified genes by comparing the expression amounts of about 1600 types of genes taken from brains of dead schizophrenic patients with those of non-schizophrenic patients.

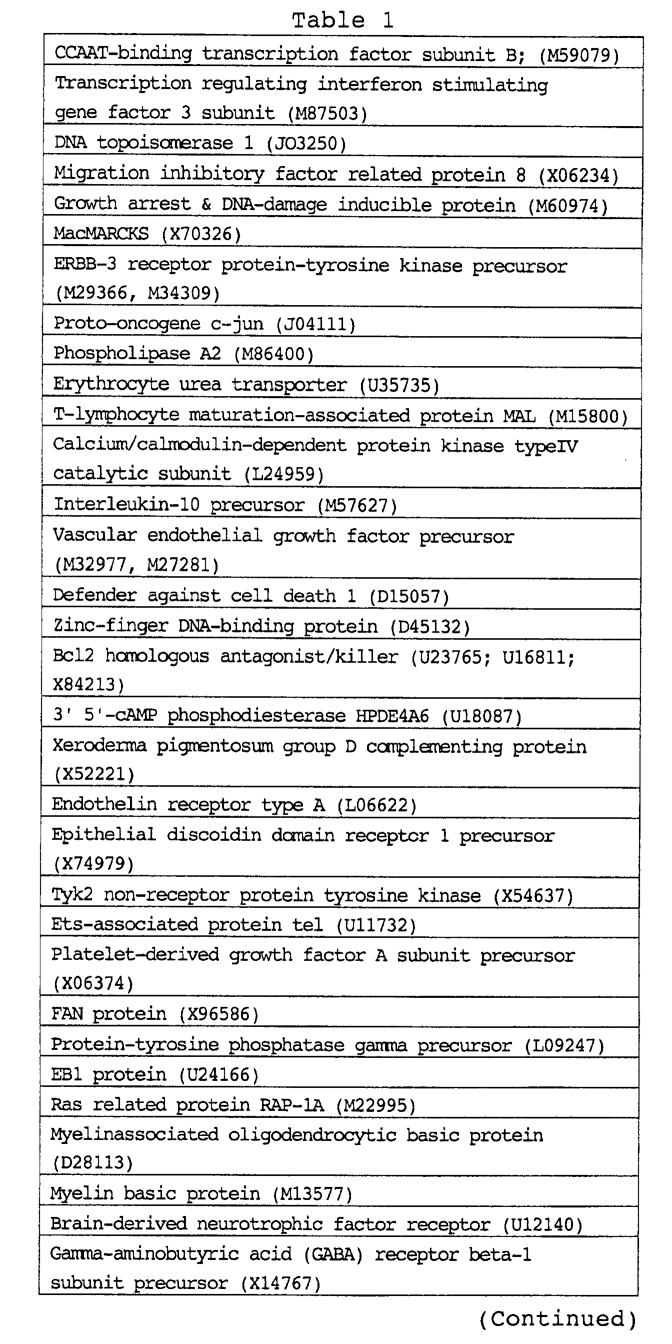

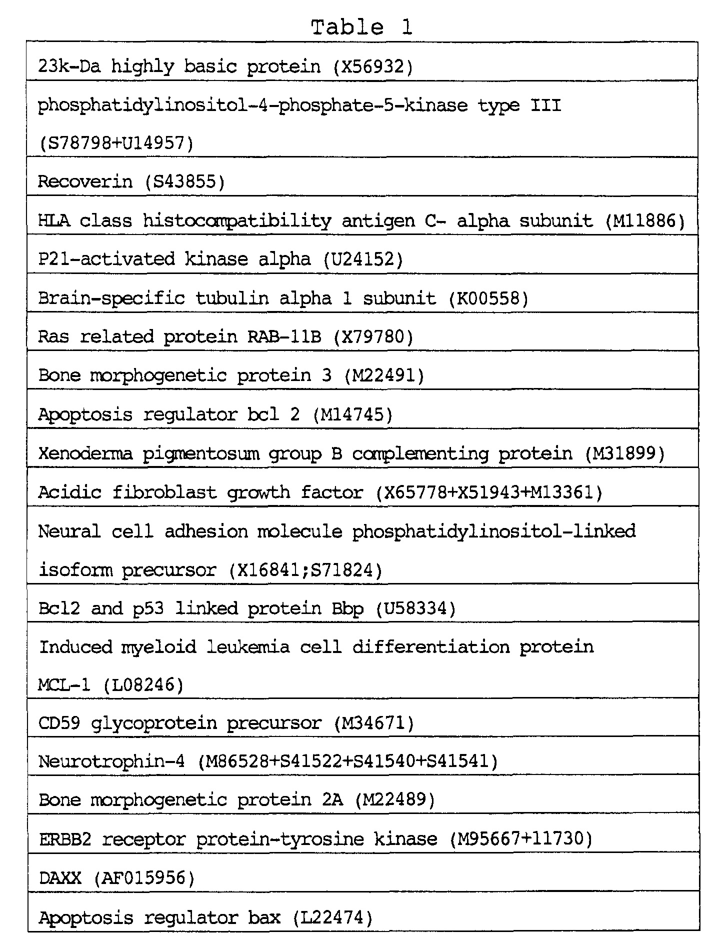

The genes (index gene) encoding the proteins listed in Table 1 have been determined to be particularly useful as a diagnostic index for schizophrenia in consideration of all of the following factors:
(1) signal intensity,
(2) a variation rate of gene-expression
   which is determined by selecting the larger one of a ratio of the patient group/the non-patient group in average expression amount and a ratio of the non-patient group/the patient group in average expression amount(see Experimental Examples), and
(3) p-value obtained by a test of difference in average expression amount of the gene between the patient group and the non-patient group.

Note that the term "p value" is a probability of measuring a certain statistical amount according to null hypothesis.

However, depending upon the accuracy required for diagnosis, the index gene may be selected based upon another standard in place of the aforementioned stringent standard (more specifically explained in "Experimental Examples").

The nucleic acid is selected either based upon the p value alone or based upon the gene-expression variation ratio alone.

When the index gene is chosen on the basis of the p value alone, the index gene preferably has the P value of 0.5 or less, more preferably 0.4 or less, more preferably 0.3 or less, more preferably 0.25 or less, more preferably 0.2 or less, more preferably 0.15 or less, more preferably 0.10 or less, and more preferably 0.05 or less. More preferably, the index gene may have the P value of 0.02 or less, 0.01 or less, 0.005 or less, 0.025 or less, or 0.001 or less.

When the index gene is selected on the basis of the gene-expression variation ratio alone, the index gene preferably has a gene-expression variation ratio of 1.1 or more, more preferably 1.2 or more, more preferably 1.25 or more, more preferably, 1.3 or more, more preferably 1.4 or more, more preferably 1.5 or more, more preferably 1.6 or more, more preferably 1.7 or more, more preferably 1.75 or more, more preferably 1.8 or more, more preferably 1.9 or more, or more preferably 2.0 or more. It is more preferable that the index gene should have a gene-expression variation ratio of 2.1 or more, 2.2 or more, 2.25 or more, 2.5 or more, 3 or more, 4 or more, 5 or more, 6 or more, 7 or more, 8 or more, 9 or more , 10 or more, 15 or more, 20 or more, 25 or more, 30 or more, 40 or more, 50 or more, 60 or more, 70 or more, 75 or more.

Depending upon the accuracy required for diagnosis, the genes listed in Table 2 below may be used as the index in place of or together with the genes listed in Table 1.

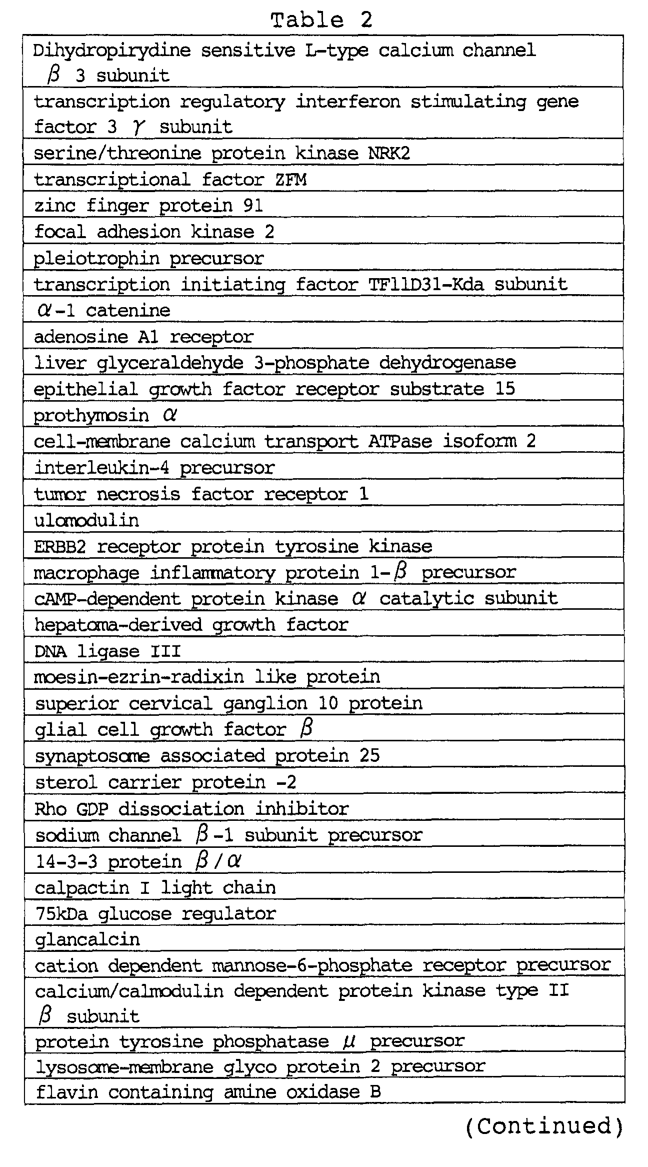

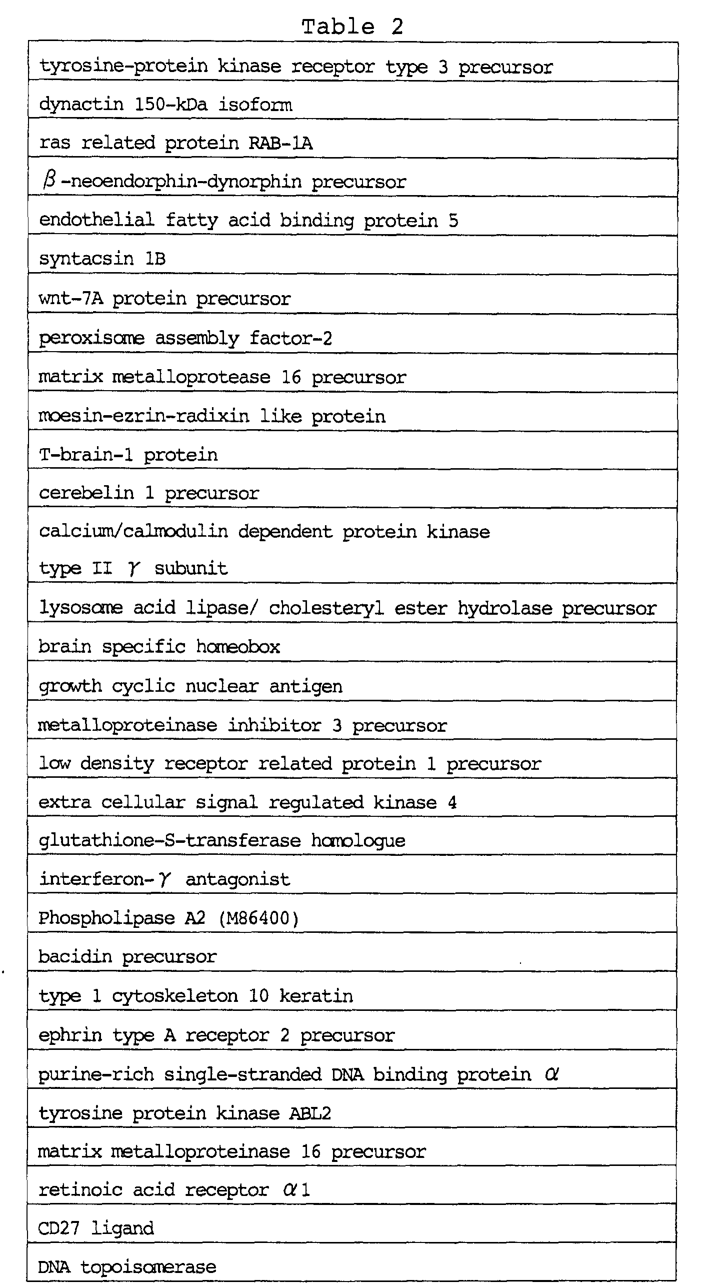

In the method of the present invention, whether or not a subject suffers from schizophrenia is objectively diagnosed on the basis of the expression amount of a gene or a fragment thereof satisfying the aforementioned standard, and/or a protein encoded by the gene or the fragment thereof.

To apply the method of the present invention, a sample containing nucleic acid or encoding said protein the protein is first taken from a subject to be checked for schizophrenia.

The term "schizophrenia" used in this text includes any type of schizophrenia such as paranoid schizophrenia, disorganized schizophrenia, catatonic schizophrenia, and schizophrenia difficult to diagnose.

In the method of the present invention, an object to be diagnosed, that is, "subject" is any mammalian animal including a human being. However, a human being is the most preferable subject.

In the method of the present invention, nucleic acid and/or protein corresponding to at least one gene selected from the group listed in Tables 1 and 2 or fragment thereof, and most preferably, the genes listed in Table 1 or fragment thereof is quantified.

Nucleic acid corresponding to genes listed in table 1 and the complementary nucleic acid thereof may generally be mRNA and cDNA of encoding the protein. Any polynucleotide such as a regulatory sequence and a polyadenyl sequence may be included in terminal ends of and/or inside a translation region of the mRNA and cDNA.

In the case where the protein listed in Table 1 is encoded by a plurality of allelic genes, all allelic genes, their transcriptional products and cDNAs thereof are included in the "nucleic acid corresponding to the genes listed in table 1 and the complementary nucleic acid thereof".

The "fragment" of the nucleic acid means polynucleotides including either whole or a part of the nucleic acid. More specifically, it means a restriction fragment of mRNA or cDNA of the protein listed in Table 1.

To quantify the expression amount of the index gene, a "sample containing nucleic-acid or protein" is taken from a subject. The nucleic acid and the protein are widely present throughout the body. As long as they are derived from the same gene, they are placed under the same control. Therefore, any sample other than the brain, taken from a subject such as tissues, cells and body fluids, can be used as the "sample containing nucleic-acid or protein". Preferable samples are a brain biopsy sample, anatomic brain, cerebrospinal fluid, or blood.

Particularly preferable samples are a biopsy sample taken from an origin or projection site of a dopaminergic neuron of the central nervous system. To be more specific, preferable samples include a biopsy sample taken from a frontal lobe, limbic system (including gyrus parahippocampalis, cingulated gyrus, gyrus subcallosus), caudatum, putamen, nucleus accumbens, amygdala, ventral tegmental decussation, and nigra.

The "nucleic acid corresponding to the genes" used herein includes any polynucleotide consisting of simple nucleotides such as cDNA, mRNA, total RNA, and hn RNA, and/or modified nucleotides. The "modified nucleotides" include phosphoric esters such as inosine, acetylcytidine, methylcytidine, methyladenosine, and methylguanosine, and other postnatal nucleotides which are possibly produced by irradiation of ultraviolet rays or application of chemical substances.

Generally, in quantifying the nucleic acid, a sample is first taken from a subject, and thereafter the nucleic acid is extracted from the sample. The nucleic acid may be extracted from the sample (biogenic sample), by any extraction method other than phenol extraction and ethanol precipitation. When a mRNA is extracted, the sample may be loaded on an oligo-dT column.

In the case where an amount of the nucleic acid is low, the nucleic acid may be amplified if necessary. The nucleic acid may be amplified by a polymerase chain reaction (hereinafter, simply referred to as "PCR"), for example, reverse transcriptase PCR (RT-PCR). Furthermore, the amplification may be performed not only to amplify the nucleic acid but also to quantify it.

After the extraction and the amplification (if necessary) are performed, nucleic acid corresponding to at least one gene listed in Table 1 or Table 2, is quantified.

The nucleic acid is quantified by any known method such as quantitative PCR, Southern blotting, Northern blotting, RNase protection mapping, or a combination thereof.

In the quantitative PCR, an amplified product may be endo-labeled by using radio-labeled (e.g., 32p) nucleotides. Alternatively, an amplified product is endo-labeled by attaching a radioactive substance to a primer and then performing an amplification reaction by use of the radio-labeled primer. Free radio-labeled nucleotides or radio-labeled primers are removed by using a known method such as gel filtration, alcohol precipitation, trichloroacetic acid precipitation, or physical adsorption using a glass filter to thereby isolate the radio-labeled amplified product. Thereafter, electrophoresis and hybridization may be applied (or may not be applied). The amplified product is then quantified by using liquid scintillation, autoradiography, and imaging plate Bio-Imaging Analyzer (BAS; Fuji Photo Film Co., Ltd.). In the case where the radioactive substance is not used, a fluorescent substance or a luminescent substance may be used as a labeling substance. In this case, the amplified product is quantified by means of a spectrofluorometer, fluoromicro plate reader, or CCD camera. Furthermore, in the case where the labeling substance is not attached to an amplified product during the PCR operation, an intercalate fluorescent pigment such as ethidium bromide, SYBR Green I™ , PicoGreen™ (manufactured and sold by Molecular Probes) may be used to detect the amplified product.

Another method may be used in place of the quantitative PCR method to quantify the nucleic acid. In this method, the nucleic-acid containing sample is subjected to electrophoresis and then analyzed by Southern blotting or Northern blotting using a probe labeled with a detectable marker.

A plurality types of nucleic acids can be quantified simultaneously if a DNA chip or a DNA microarray is used together with or in place of the aforementioned methods.

The expression amount of a gene (nucleic acids) may be indirectly determined by quantifying the protein encoded by the nucleic acids (gene). The indirect quantification method may be used together with the nucleic acid quantification method. The indirect method which quantifies a protein, may, in most cases, be more useful than the direct quantification method for nucleic acid when schizophrenia is diagnosed in accordance with the method of the present invention. Even if the expression amount of certain nucleic acid (gene) of a schizophrenic patient differs from that of a non-schizophrenic patient, as described in Example 2, the amount of the protein encoded by the nucleic acid does not differ in some cases. In some cases, the amount of the protein is high whereas the expression amount of the nucleic acid is low. Therefore, in the indirect method (of determining the expression amount of the nucleic acid by quantifying the protein), it is desirable to previously check whether the target protein is present in a larger amount or in a smaller amount in a schizophrenic patient than in a non-schizophrenic patient.

As a method of extracting the protein from tissues and a method of quantifying the protein, any methods are used as long as they are known in this field. Examples of protein quantification methods include Western blotting and enzyme-linked immunosorbent assay such as a solid-phase enzyme-linked immunosorbent assay, immunocytochemistry, and immunohistochemistry.

In this text, the most usually employed method is schematically exemplified. The aforementioned method may be modified in various ways. Furthermore, a completely different method may be used.

The extraction, amplification, isolation, and quantification of the nucleic acid may be automatically performed by use of an automatic operation machine currently on the market, in which an electrophoresis unit, a PCR unit and the like are installed. If the automatic machine is used, diagnosis of schizophrenia can be made in the same procedure as in routinely performed clinical tests.

After a predetermined nucleic acid and/or protein is quantified, whether or not the subject suffers from schizophrenia is determined based upon the quantitative value as an index.

When diagnosis is made by using a single quantitative value of nucleic acid and/or protein as the index, a threshold value is first set appropriately with reference to a normal value. Then, if the quantitative value is higher or lower than the threshold value, there is a high possibility that the subject suffers from schizophrenia. For example, when the quantitative value is high in schizophrenic patients, if the quantitative value is higher than a predetermined threshold, it is determined that it is highly possible that the subject suffers from schizophrenia.

The threshold value may be selected depending upon how accurately diagnosis is made, as shown below.

When the distribution of gene expression amount is known in both the non-schizophrenic group (normal group) and the schizophrenic group (patient group), the threshold may be determined such that the subject belongs to the non-schizophrenic group with a probability of 10%, 5% or 1%.

When the distribution of the gene expression amount is known only in the non-schizophrenic group, the assumption is made that the subject (from which the target nucleic acids or protein is taken) belongs to the non-schizophrenic group. Under this assumption, the threshold (of nucleic acid or protein in amount or concentration) may be determined such that the subject belongs to the non-schizophrenic group with a probability (p-value, typically two-way possibility, a one-way possibility) of 10%, 5%, or 1%.

On the other hand, when the distribution of the gene expression amount is known only in the patient group, analysis can be made in the same manner using a statistical method.

The p-value can be obtained by a statistical test such as the t-test or a non-parametric test.

To obtain a reliable statistical distribution of gene expression amount of the normal group and the patient group, at least 5 individuals, preferably 10 individuals, more preferably, 20 individuals, further preferably, 50 individuals, and most preferably, 100 individuals are generally required to be measured.

Whether or not a subject suffers from schizophrenia can be determined more accurately by another statistic method. This diagnostic method using such a statistic method should fall within the scope of the present invention.

In the case where diagnosis is made based on a single index value selected from the quantitative values of the nucleic acid and protein corresponding to the gone, it is preferable that the quantitative value to be used as the index should satisfy the following conditions.
1) The quantitative value to be used as the index is large either in the normal group or in the patient group (10 or more signals, see "Experimental Examples").
2) The quantitative value differs by 1.4 times or more between both groups (see Experimental Examples).
3) The quantitative value gives a p value of 5% or less in the test of mean-value difference.

When the diagnosis is made by using a plurality of quantitative values of nucleic acid and/or protein corresponding to the gene, an appropriate threshold is set with respect to each of the nucleic acid and protein corresponding to the genes. The diagnosis is made by checking that the expression amount is higher or lower than the threshold with respect to individual genes, in the same manner as when the single index is used.

If one of the quantitative values of nucleic acid and protein is higher or lower than the threshold, it is possible to determine that the subject may suffer from schizophrenia.

If at least two quantitative values of nucleic acid and protein are higher or lower than the thresholds, respectively, there is a further high possibility that the subject suffers from schizophrenia. The more the number of nucleic acid and/or proteins is used, the more accurately the diagnosis is made. Therefore, the number of quantitative values may be chosen depending upon a desired accuracy of diagnosis.

The diagnostic method of the present invention can be used together with the conventional objective diagnostic method.

On the other hand, if quantitative data for nucleic acid and/or protein to be used as an index in the diagnostic method of the present invention can be obtained from patients clearly suffering from schizophrenia (determined in some way), it is possible to determine schizophrenia, without fail by the method of the present invention alone.

The subject of the present invention resides in providing an objective diagnostic method for schizophrenia but does not reside in individual extractions, amplifications, and analytic operations specifically described in the text. Hence, it should be noted that the diagnostic methods other than the aforementioned operations are also included in the present invention.

As described in the above, if the method of the present invention is used, whether or not a subject suffers from schizophrenia can be objectively diagnosed by using the expression amount of nucleic acid (gene) and biological product (protein) derived from the nucleic acid (gene) as an index.

Therefore, the method of the present invention is further applicable to a method for selecting a useful schizophrenic animal model (excluding human beings) and to a method for evaluating efficacy of the drug in a drug screening test using such an animal model.

Usefulness of the schizophrenic animal model is determined in the same manner as the diagnostic method. More particularly, whether or not the animal subject suffers from schizophrenia is first determined on the basis of the expression amount of a predetermined gene. Then, if the animal subject suffers from schizophrenia, the animal is determined useful as a schizophrenic animal model.

Examples of the animal subject include mice, rats and monkeys. Any animal can be employed as the animal subject as long as the animal is not a human being.

Since it has been more difficult to diagnose schizophrenia of animals than human beings, this method is extremely useful.

Furthermore, after a possible anti-schizophrenia drug is administered to the animal model, the amount of predetermined nucleic acid and/or protein is determined as described above. If the animal recovers from schizophrenia or the schizophrenic condition of the animal is improved, it may be safe to determine that the possible drug has an anti-schizophrenic efficacy. Hence, if the diagnostic method of the present invention is used, screening of the possible anti-schizophrenic drug can be made easily and accurately.

Any substance may be employed as the "possible anti-schizophrenic drug".

The diagnostic method of the present invention can be also applied to a psychiatric assessment to check whether or not a subject is legally responsible and to a psychiatric assessment performed for another purpose.

Now, the present invention will be further explained in detail with reference to Experimental Examples and Examples, which will not limits the scope of the present invention in any sense.

### [Experimental Example 1]

In this experimental example 1, we will explain the genes identified by the present inventors as a possible diagnostic index.

In this experiment, an RNA was extracted from tissues of frontal lobes (brain) of dead schizophrenic patients (Sample group S1) and non-schizophrenic patients (Sample group S2). Thereafter, the quality of the extracted RNAs was checked.

Six or three RNA samples are selected from the RNAs qualified good for each group. Then, DNAs were amplified by using RNAs (Total 12 or 6), a reverse transcriptase, and a radioactive phosphorus label. The resultant radio-labeled DNA is used as a probe, which is applied to three types of DNA microarrays (manufactured and sold by Clonetech). In this method, the expression amounts of a plurality of genes are simultaneously measured, at the same time, patterning (molecular expression profile) of the genes can be made.

The three types of DNA microarrays used herein are Atras human cDNA expression array, Atras human neurobiology array, and Atras human cancer cDNA expression array.

The deposition numbers (Q7) of the genes at GenBank are attached onto individual microarrays. The deposition numbers are listed in Table 3.

The signals for individual gene spots were measured and quantified by BAS5000 image analyzer (Fuji Photo Film Co., Ltd.). In order to correct variation of signal intensities between DNA microarray sheets which correspond to individual RNA samples, the signal intensity is standardized assuming that the sum of all gene expression signals on any sheet is constant even if the sample RNA differs.

To identify a gene commonly observed in a plurality of schizophrenic patients and exhibiting a specific change in expression amount, analysis is made on data of the expression signals obtained from the schizophrenic patients (Sample group S1, N = 6 or 3) and from non-schizophrenic patients (Sample group S2, N = 6 or 3). The expression signals of the same gene are obtained from two spots in order to obtain a more accurate value.

The signal data were analyzed by using a significant difference test according to the student t-test. The results are shown in Table 3 below.

In this experimental example, in the case of the genes of the S1 group and S2 group having an average signal intensity larger than 10, if a gene has
1) a gene-expression variation ratio (larger one selected from a ratio of the S1 group/the S2 group in average expression amount or a ratio of the S2 group/the S1 group in average expression amount) of 1.4 or more, and
2) a P value of 0.05 or less,
   the expression amount of the gene is determined as being changed significantly due to schizophrenia.

In the case of the genes having an average signal intensity of 10 or less, if a gene has
1) an expression variation rate of 1.8 or more, and
2) a P value is 0.01 or less,
   the expression amount of the gene is determined as being changed significantly due to schizophrenia.

Hence, the genes listed in Table 1 selected on the basis of the aforementioned standards are particularly useful as a diagnostic index of schizophrenia.

**Table 3**

| Genaral | | | Neurobiology | | | Cancer | | |
|---|---|---|---|---|---|---|---|---|
| GenBank No. | p value | variation rate | GenBank No. | p value | variation rate | GenBank No. | p value | variation rate |
| X70326 | 0.000 | 1.70 | L15189 | 0.002 | 1.25 | L22474 | 0.000 | 2.57 |
| U24166 | 0.000 | 1.81 | U50358 | 0.002 | 1.21 | M34671 | 0.000 | 1.40 |
| M87503 | 0.000 | 2.49 | M13577 | 0.002 | 1.57 | L20471 | 0.001 | 1.17 |
| X52221 | 0.001 | 4.08 | D28113 | 0.004 | 1.69 | M86400 | 0.001 | 1.21 |
| D15057 | 0.001 | 1.43 | M58583 | 0.004 | 1.34 | U90313 | 0.001 | 1.26 |
| J03250 | 0.002 | 2.18 | M22995 | 0.004 | 2.46 | L08246 | 0.002 | 1.57 |
| X96586 | 0.003 | 2.00 | X79780 | 0.006 | 1.41 | M95667+ M11730 | 0.003 | 5.47 |
| U23765; U16811; X84213 | 0.003 | 33.8 | M75883 | 0.006 | 1.33 | D25216 | 0.003 | 1.68 |
| U14188 | 0.003 | 121.4 | X14767 | 0.006 | 1.47 | M22489 | 0.004 | 8.61 |
| M21121 | 0.003 | 1.22 | 578798+ U14957 | 0.007 | 1.44 | X65778+ X51943+ M13361 | 0.005 | 2.23 |
| X06374 | 0.005 | 2.15 | U12140 | 0.008 | 1.51 | M63099 | 0.005 | 18.4 |
| L12261 | 0.006 | 10.1 | Z18956 | 0.008 | 2.97 | AF015956 | 0.008 | 3.30 |
| L25124; D28472 | 0.007 | 5.22 | M11886 | 0.009 | 1.44 | U58334 | 0.008 | 1.67 |
| M31165 | 0.008 | 3.96 | X07767 | 0.013 | 1.12 | K03214; X03996 | 0.008 | 4.57 |
| L29220 | 0.009 | 5.09 | L10338 | 0.013 | 1.31 | M14745 | 0.009 | 2.53 |
| U18087 | 0.009 | 4.95 | S82024 | 0.014 | 1.37 | M86528+ S41522+ S41540+ S41541 | 0.011 | 13.4 |
| L49207+ U43522+ U33284 | 0.009 | 1.33 | D37933 | 0.018 | 1.50 | X13916 | 0.013 | 1.30 |
| X74979 | 0.009 | 3.04 | X56932 | 0.021 | 1.45 | Z30183 | 0.014 | 1.34 |
| M29366; M34309 | 0.010 | 1.69 | X58288 | 0.022 | 1.21 | D50477 | 0.016 | 2.26 |
| L19067 | 0.010 | 3.24 | M94856 | 0.023 | 1.65 | U49262+ U75651 | 0.016 | 1.63 |
| U39657 | 0.010 | 26.3 | M81457 | 0.023 | 1.26 | M96684 | 0.018 | 3.61 |
| X06234 | 0.010 | 2.16 | S77512 | 0.025 | 2.85 | X59727 | 0.018 | 1.28 |
| L09247 | 0.010 | 1.92 | D17517 | 0.025 | 1.19 | X07876 | 0.019 | 4.08 |
| U11732 | 0.011 | 2.35 | X86809 | 0.026 | 1.15 | M35296 | 0.020 | 2.45 |
| X54637 | 0.011 | 2.48 | M69177 | 0.026 | 1.20 | M19156 | 0.021 | 6.45 |
| M60974 | 0.012 | 1.80 | M28209 | 0.028 | 1.16 | M15796; J04718 | 0.021 | 1.36 |
| M26708 | 0.013 | 1.20 | S43855 | 0.028 | 1.44 | X16841; S71824 | 0.021 | 1.83 |
| L06622 | 0.013 | 3.24 | M81637 | 0.030 | 1.24 | U91985 | 0.023 | 5.37 |
| D16431 | 0.013 | 2.73 | K00558 | 0.030 | 1.41 | M31899 | 0.024 | 2.47 |
| M33294 | 0.014 | 3.49 | D50477 | 0.030 | 1.43 | U43148 | 0.024 | 26.1 |
| M22489 | 0.016 | 4.10 | U50359 | 0.031 | 1.33 | U07418 | 0.028 | 922 |
| J04111 | 0.016 | 1.66 | M74775 | 0.033 | 1.25 | AB000220 | 0.029 | 5.03 |
| S64045 | 0.016 | 4.83 | L11353; Z22664; X72657; L27133 | 0.034 | 1.37 | J04088 | 0.029 | 1.89 |
| D26120 | 0.017 | 1.34 | U56602 | 0.035 | 1.44 | M59371 M36395 | 0.030 | 5.21 |
| M59079 | 0.017 | 2.72 | M84739 | 0.035 | 2.47 | A25270 | 0.031 | 1.21 |
| U07236 | 0.018 | 8.25 | M16985 | 0.036 | 1.23 | D25303; L24158 | 0.032 | 11.2 |
| J04130 | 0.018 | 2.96 | X69550 | 0.037 | 1.31 | M38690 | 0.033 | 1.47 |
| M17778 | 0.018 | 3.43 | M23725 | 0.038 | 1.12 | X07819 | 0.035 | 7.65 |
| M81840 | 0.019 | 18.0 | U49250 | 0.038 | 1.35 | AF010316 | 0.041 | 3.14 |
| L16464 | 0.020 | 2.94 | A26792 | 0.040 | 2.55 | M22491 | 0.042 | 9.08 |
| S56143 | 0.020 | 1.25 | L19761 | 0.041 | 1.33 | X85133 | 0.044 | 19.6 |
| D13866; D14705; L23805; L22080 | 0.020 | 1.28 | M14745 | 0.041 | 1.32 | L08096; S69339 | 0.045 | 2.15 |
| M63618 | 0.020 | 5.27 | X98801 | 0.041 | 1.18 | X06538; X06614+ M73779 | 0.045 | 2.24 |
| U07139 | 0.022 | 1.39 | K02268 | 0.041 | 2.07 | L13698 | 0.048 | 9.06 |
| M86400 | 0.022 | 1.63 | U24152 | 0.043 | 1.42 | U66406 | 0.051 | 2.99 |
| L20977 | 0.023 | 8.28 | D14838 | 0.044 | 1.76 | L25080 | 0.051 | 1.17 |
| D45132 | 0.023 | 1.43 | U53476 | 0.045 | 1.48 | U14588 | 0.052 | 1.72 |
| L20321 | 0.023 | 1.37 | J04183 | 0.046 | 1.21 | Z70519 | 0.052 | 1.80 |
| L24959 | 0.024 | 1.54 | X55758 | 0.046 | 4.09 | U29680; Y09397 | 0.056 | 3.29 |
| L07540 | 0.024 | 12.2 | X57346 | 0.048 | 1.29 | X04434; M24599 | 0.057 | 3.00 |
| M31145 | 0.026 | 1.64 | Z21966 | 0.048 | 1.21 | X53655; M37763 | 0.057 | 2.27 |
| L11672 | 0.028 | 1.34 | M86492 | 0.049 | 1.35 | L12260; L12261+ U02326+ M94165 | 0.060 | 2.60 |
| L11353; Z22664; X72657; L27133 | 0.029 | 2.19 | Z18954 | 0.050 | 2.35 | U02687 | 0.060 | 7.07 |
| M57627 | 0.030 | 1.50 | U00802 | 0.050 | 1.24 | M28249; X17033 | 0.065 | 2.46 |
| U17075; L36844 | 0.030 | 1660 | X68486 | 0.051 | 197 | M65291 | 0.065 | 2.36 |
| L04791 | 0.031 | 8.19 | L36870 | 0.053 | 1.14 | M21616 | 0.067 | 1.94 |
| M15800 | 0.033 | 1.57 | U11053 | 0.053 | 8.35 | M68520 | 0.068 | 2.46 |
| M80634+ U11814+ X52832; M35718+ M87771+ M87772 | 0.034 | 1.72 | X82676 | 0.053 | 3.57 | M81934; S78187 | 0.069 | 1.90 |
| X52946+ D90226+ M57399 | 0.035 | 1.32 | L12392 | 0.053 | 1.21 | U69108 | 0.069 | 4.44 |
| U30504 | 0.036 | 1.29 | M28212 | 0.054 | 1.25 | J00209; J00207 | 0.071 | 10.7 |
| L07032 | 0.037 | 19.1 | S82769 | 0.055 | 1.63 | D11117 | 0.072 | 8.21 |
| M32977; M27281 | 0.038 | 1.49 | X97064 | 0.055 | 1.30 | X91940 | 0.073 | 1.57 |
| X00588; K03193; X00663; U48722 | 0.040 | 2.83 | M25667 | 0.058 | 1.22 | M90813+ D13639 | 0.073 | 1.44 |
| U61262 | 0.041 | 1.28 | U93703 | 0.062 | 2.02 | X65923 | 0.073 | 2.23 |
| U35735 | 0.042 | 1.58 | U95020 | 0.062 | 2.07 | X60592 | 0.074 | 2.87 |
| X84740 | 0.042 | 2.29 | M55047 | 0.063 | 1.43 | U18291 | 0.078 | 1.74 |
| X07767 | 0.044 | 2.95 | D16111 | 0.065 | 1.12 | M19154; M22045; M22046; Y00083 | 0.080 | 1.25 |
| L36052; L36051; U11025 | 0.044 | 3.74 | M22430; J04704 | 0.065 | 1.29 | M61176 | 0.080 | 5.76 |
| U22398 | 0.045 | 3.26 | D31840 | 0.066 | 1.61 | U91903+ U24163+ U68057 | 0.083 | 2.58 |
| U33635+ U40271 | 0.045 | 7.72 | X90840 | 0.068 | 1.60 | M62402 | 0.083 | 2.64 |
| D14520 | 0.046 | 1.92 | M31724 | 0.068 | 1.23 | M63959 | 0.083 | 2.27 |
| M13982 | 0.046 | 5.13 | M90359 | 0.068 | 1.18 | U60520; U58143; X98172; X98173; X98174; AF00962 | 0.084 | 2.87 |
| X04571 | 0.047 | 176 | M68956 | 0.069 | 1.28 | L38734 | 0.086 | 1.88 |
| X17543; M30134 | 0.048 | 4.08 | U50040 | 0.071 | 1.35 | Z71621 | 0.086 | 10.8 |
| M95667+ M11730 | 0.049 | 3.20 | L34339 | 0.072 | 2.71 | U77493 | 0.087 | 8.13 |
| X89986; U34584 | 0.052 | 6.96 | M81778 | 0.072 | 123 | X79483 | 0.087 | 2.03 |
| X52541; M62829 | 0.053 | 1.56 | D10495 | 0.073 | 1.58 | U12535 | 0.088 | 5.39 |
| U08098 | 0.055 | 2.49 | M81883 | 0.075 | 1.38 | X95425 | 0.090 | 3.24 |
| X06233 | 0.055 | 1.70 | M58026 | 0.076 | 2.03 | M61916 | 0.091 | 11.4 |
| L20320 | 0.055 | 23.7 | M75126 | 0.079 | 1.15 | X59798; M64349 | 0.091 | 1.96 |
| K00558 | 0.056 | 1.72 | Z15114 | 0.080 | 1.80 | X17543; M30134 | 0.092 | 4.77 |
| X56932 | 0.057 | 1.45 | S62908 | 0.080 | 1.41 | X85960 | 0.096 | 1.27 |
| M11886 | 0.058 | 1.90 | X01677 | 0.081 | 1.17 | D17517 | 0.097 | 1.09 |
| X74764 | 0.058 | 2.52 | U19721 | 0.081 | 1.17 | U25265 | 0.097 | 1.30 |
| M81750 | 0.059 | 3.16 | D38293 | 0.084 | 1.73 | U12431+ U29943 | 0.098 | 1.45 |
| X90392; L40817; U06846 | 0.059 | 2.38 | U78107 | 0.084 | 1.97 | Y09392+ U75380+ U74611+ U83597 | 0.099 | 1.53 |
| U20536+ U20537 | 0.059 | 56.3 | M30773 | 0.087 | 1.14 | X52221 | 0.100 | 3.80 |
| M28622 | 0.064 | 4.88 | U10554 | 0.091 | 2.30 | S78085 | 0.100 | 1.17 |
| X13967; M63420 | 0.065 | 3.47 | U32315 | 0.093 | 1.22 | L42379 | 0.101 | 3.74 |
| D21878 | 0.065 | 5.14 | X74764 | 0.094 | 11.2 | M62880; S80335 | 0.103 | 1.46 |
| S59184 | 0.065 | 1.57 | M29366; M34309 | 0.094 | 1.78 | M34065 | 0.105 | 3.77 |
| U04897+ L14611 | 0.066 | 2.06 | X15804 | 0.096 | 1.53 | M31213+ M57464 | 0.108 | 2.89 |
| J04088 | 0.068 | 1.90 | U54644 | 0.101 | 1.12 | Z35227 | 0.109 | 2.76 |
| X01677 | 0.069 | 1.23 | X70904; X91171 | 0.101 | 1.51 | Y10479 | 0.111 | 1.50 |
| M15990 | 0.072 | 1.98 | M59371M 36395 | 0.101 | 2.30 | AF001954 | 0.112 | 2.10 |
| M88163 | 0.073 | 1.45 | M57730; M37476 | 0.102 | 1.63 | M11886 | 0.112 | 1.39 |
| L06801 | 0.075 | 4.46 | L34155 | 0.103 | 1.80 | X95282 | 0.115 | 1.78 |
| U07707; Z29064 | 0.076 | 1.21 | L05624 | 0.106 | 1.19 | X98085 | 0.120 | 1.19 |
| L25080 | 0.076 | 1.12 | D16826 | 0.107 | 1.40 | M26326 | 0.120 | 2.34 |
| M57230 | 0.076 | 1.55 | X00351 | 0.109 | 1.18 | X06256 | 0.122 | 2.68 |
| M97191 | 0.076 | 2.33 | X77197 | 0.110 | 1.23 | M32977; M27281 | 0.123 | 1.59 |
| U14407 | 0.076 | 4.13 | M14694; M14695 | 0.112 | 1.94 | J03241 | 0.125 | 2.11 |
| X66945; M34641; M34186; M37722+ M63887+ M63888+ M63889 | 0.076 | 1.32 | U25278 | 0.113 | 1.52 | M21772; M20336 | 0.127 | 1.93 |
| L29511; M96995 | 0.077 | 1.19 | M15182 | 0.113 | 1.41 | M60828 | 0.129 | 39.3 |
| M34960 | 0.079 | 3.37 | M29066 | 0.113 | 98.2 | M94151 | 0.129 | 2.18 |
| U14722 | 0.080 | 1.72 | M98529 | 0.114 | 1.13 | X79981; X59796 | 0.129 | 2.24 |
| L12260; L12261+ U02326+ M94165 | 0.080 | 4.02 | M83941 | 0.115 | 2.45 | Y10256 | 0.130 | 1.39 |
| M65291 | 0.081 | 4.27 | L33075 | 0.117 | 1.20 | M81104 | 0.132 | 2.42 |
| M68891 | 0.083 | 1.77 | S87759 | 0.123 | 1.06 | X03124 | 0.134 | 4.89 |
| M29038 | 0.084 | 5.32 | U07819 | 0.125 | 1.26 | U66469 | 0.136 | 1.23 |
| M57502 | 0.087 | 2.47 | U25265 | 0.125 | 1.11 | J03171 | 0.137 | 1.53 |
| M29971 | 0.087 | 2.21 | X54871 | 0.126 | 1.12 | X94991; X95735 | 0.138 | 2.07 |
| U28838 | 0.090 | 1.96 | M64930 | 0.126 | 1.12 | M54995; M38441 | 0.138 | 1.71 |
| K03515 | 0.092 | 1.80 | M61900 | 0.129 | 1.40 | U43318 | 0.138 | 17.9 |
| D26121 | 0.093 | 1.25 | X70218 | 0.129 | 1.69 | L34059 | 0.140 | 2.14 |
| X12795; X16155; X58241 | 0.093 | 1.14 | M68840 | 0.130 | 1.30 | M34225 | 0.140 | 1.82 |
| M60915 | 0.093 | 2.54 | M97252 | 0.130 | 1.20 | U59747 | 0.140 | 2.00 |
| L29216 | 0.095 | 2.54 | D00017 | 0.134 | 1.47 | X13967; M63420 | 0.140 | 1.97 |
| M74777 | 0.095 | 2.78 | L09229 | 0.135 | 1.39 | X00588; K03193; X00663; U48722 | 0.142 | 1.47 |
| M96684 | 0.095 | 2.10 | L13939 | 0.138 | 1.33 | X66362 | 0.145 | 1.60 |
| M73812 | 0.096 | 3.70 | L02752 | 0.138 | 1.21 | X74594 | 0.148 | 2.19 |
| M24898 | 0.096 | 2.04 | M12625 | 0.139 | 1.92 | M97935 | 0.155 | 1.65 |
| M14743; M17115 | 0.097 | 3.09 | U92459 | 0.140 | 2.36 | M74524 | 0.158 | 1.31 |
| J00209; J00207 | 0.097 | 6.30 | X85545 | 0.140 | 2.03 | L20688 | 0.158 | 1.87 |
| J05081 | 0.097 | 2.72 | M64929 | 0.142 | 1.10 | U56976 | 0.158 | 1.49 |
| U03688 | 0.098 | 3.87 | L35253; L35263 | 0.142 | 1.18 | U01038 | 0.160 | 1.82 |
| M97796 | 0.102 | 1.70 | U62438 | 0.144 | 2.82 | X02851 | 0.160 | 1.76 |
| U10564 | 0.105 | 3.58 | X74008 | 0.145 | 1.15 | U43746 | 0.160 | 4.41 |
| M60278 | 0.107 | 1.69 | X74837 | 0.147 | 1.77 | U25278 | 0.161 | 2.50 |
| M30257 | 0.107 | 7.60 | M22960 | 0.147 | 1.15 | X03663 | 0.162 | 3.47 |
| M16937 | 0.108 | 1.81 | U37352 | 0.148 | 1.18 | X14787 | 0.162 | 190 |
| M25667 | 0.108 | 1.57 | U36269 | 0.148 | 3.54 | X83929+ D17427 | 0.162 | 2.71 |
| U84119 | 0.110 | 5.16 | M64788 | 0.148 | 3.82 | U49070 | 0.163 | 1.23 |
| A00914 | 0.114 | 2.59 | X80693 | 0.148 | 1.17 | X97442 | 0.163 | 1.29 |
| J03132 | 0.117 | 2.15 | M62400 | 0.148 | 5.12 | X51688 | 0.163 | 1.97 |
| M54992 | 0.117 | 2.96 | M26880 | 0.150 | 1.14 | M30938 | 0.163 | 2.09 |
| M28215 | 0.118 | 1.17 | M55514 | 0.156 | 1.57 | X75308 | 0.164 | 1.84 |
| L11015 | 0.119 | 5.15 | M82919 | 0.158 | 1.32 | X78817 | 0.168 | 1.51 |
| M75952 | 0.119 | 6.07 | X16937 | 0.159 | 1.22 | U54777 | 0.169 | 1.81 |
| X15722 | 0.120 | 5.08 | X97370 | 0.164 | 3.09 | X85134 | 0.170 | 1.28 |
| L34673 | 0.121 | 1.31 | X06389 | 0.165 | 1.25 | L22548 | 0.170 | 2.93 |
| L07868 | 0.124 | 1.56 | M28211 | 0.166 | 1.32 | U15979; Z12172 | 0.173 | 2.98 |
| X68742 | 0.125 | 2.76 | X93921 | 0.166 | 1.16 | L35253; L35263 | 0.174 | 1.27 |
| Y00796 | 0.126 | 1.16 | U27831 | 0.167 | 1.10 | AF010310; AF010311 | 0.175 | 1.90 |
| M76766 | 0.131 | 1.16 | X52836 | 0.168 | 1.24 | L34075 | 0.176 | 4.25 |
| U66838 | 0.132 | 1.29 | U77942 | 0.168 | 1.13 | M60315 | 0.176 | 204 |
| M60459 | 0.135 | 2.10 | X76132 | 0.169 | 1.30 | U36223 | 0.179 | 1.45 |
| X13293 | 0.136 | 1.98 | M65128 | 0.169 | 1.12 | U94352 | 0.179 | 4.83 |
| M96944 | 0.138 | 2.03 | L19058 | 0.171 | 2.06 | J04177 | 0.181 | 2.26 |
| U08839; M83246; X51675 | 0.140 | 3.31 | M30269 | 0.171 | 1.87 | X89576 | 0.183 | 1.20 |
| X51602+ U01134 | 0.140 | 1.62 | X63465 | 0.172 | 1.37 | X16468 | 0.184 | 77.7 |
| D13318 | 0.140 | 1.26 | M55040 | 0.173 | 1.20 | M25639 | 0.184 | 1.18 |
| X01060 | 0.141 | 1.95 | D73409 | 0.174 | 1.18 | Z29083 | 0.184 | 5.65 |
| M24545 | 0.141 | 2.20 | L28957 | 0.176 | 1.15 | AF003521 | 0.186 | 1.61 |
| M13194 | 0.142 | 1.36 | U16127 | 0.177 | 7.90 | K00065; X02317 | 0.192 | 1.17 |
| U15979; Z12172 | 0.142 | 4.47 | M81829 | 0.177 | 1.97 | U32907 | 0.195 | 3.76 |
| X54469; M28019 | 0.146 | 1.75 | M74387 | 0.178 | 1.12 | U07695 | 0.196 | 12.1 |
| U02619 | 0.149 | 3.51 | X56351 | 0.181 | 1.19 | X05232 | 0.197 | 3.06 |
| X01992; M29383 | 0.150 | 3.32 | M21054 | 0.182 | 1.16 | S77512 | 0.201 | 15.7 |
| X60188 | 0.151 | 1.23 | X75208 | 0.182 | 3.25 | M91196 | 0.202 | 1.97 |
| L15344 | 0.151 | 3.44 | U18009 | 0.183 | 1.19 | X06820 | 0.204 | 1.31 |
| M93255 | 0.151 | 3.60 | U29171 | 0.183 | 1.08 | U47414; L49506 | 0.206 | 2.21 |
| X01394 | 0.152 | 365 | X53179 | 0.184 | 1.19 | M29366; M34309 | 0.206 | 1.70 |
| X83441 | 0.152 | 1.20 | L07032 | 0.184 | 1.82 | X66363 | 0.207 | 2.19 |
| M25627 | 0.152 | 3.90 | X79781 | 0.184 | 1.13 | A03911 | 0.208 | 1.42 |
| L08096; S69339 | 0.153 | 114 | X79483 | 0.187 | 1.65 | U23435; U31089 | 0.209 | 1.37 |
| S85655; U17179 | 0.155 | 1.74 | L01439 | 0.191 | 1.31 | M14743; M17115 | 0.212 | 2.02 |
| D10923 | 0.158 | 153 | U61538 | 0.195 | 1.14 | U24166 | 0.212 | 1.27 |
| D21235 | 0.159 | 1.23 | X60188 | 0.197 | 1.11 | S40706; S62138 | 0.213 | 1.49 |
| U02082 | 0.159 | 7.09 | U32680 | 0.198 | 1.22 | Z18951; S49856 | 0.213 | 3.48 |
| M83554 | 0.160 | 2.54 | M13667 | 0.202 | 1.12 | X57766 | 0.215 | 1.48 |
| M65290 | 0.161 | 2.62 | U07882 | 0.203 | 294 | X56932 | 0.215 | 1.19 |
| D30751+ M22490 | 0.161 | 2.17 | U14187 | 0.203 | 1.18 | U52111 | 0.220 | 2.62 |
| X86779 | 0.161 | 1.28 | S78873 | 0.204 | 1.39 | X53586; X59512 | 0.228 | 1.94 |
| U39613 | 0.162 | 2.01 | M13520 | 0.204 | 1.36 | X67683 | 0.229 | 4.43 |
| L20433 | 0.164 | 3.39 | X65293 | 0.206 | 1.30 | U46010 | 0.231 | 3.67 |
| U03494 | 0.169 | 1.18 | X81197 | 0.207 | 1.10 | M27968 | 0.231 | 1.18 |
| M14631 | 0.169 | 1.09 | U71127 | 0.210 | 1.54 | M63618 | 0.231 | 2.28 |
| U06863 | 0.170 | 1.88 | M18377 | 0.210 | 1.16 | S82185 | 0.231 | 927 |
| M68932 | 0.170 | 2.72 | X97867 | 0.211 | 1.59 | M19722 | 0.233 | 1.68 |
| U02326 | 0.171 | 1.48 | X80910 | 0.212 | 1.07 | L22005 | 0.238 | 1.07 |
| X03663 | 0.171 | 1.48 | J05633 | 0.213 | 1.58 | X01992; M29383 | 0.239 | 2.36 |
| U26710 | 0.173 | 1.64 | J05401 | 0.216 | 1.63 | U02570 | 0.239 | 1.46 |
| X04688; J03478 | 0.173 | 2.39 | Y08200 | 0.217 | 2.09 | X89986; U34584 | 0.240 | 3.76 |
| U41816 | 0.174 | 1.17 | M27545; X06318 | 0.218 | 1.12 | U13699; M87507; X65019 | 0.245 | 1.64 |
| M33374 | 0.175 | 1.21 | L40636 | 0.218 | 119 | M30269 | 0.245 | 2.53 |
| U05340 | 0.175 | 2.41 | L25541 | 0.220 | 1.63 | U94354 | 0.247 | 7.13 |
| D28538 | 0.175 | 1.41 | U83192 | 0.221 | 1.20 | Y00503 | 0.249 | 1.94 |
| M92299 | 0.177 | 40.1 | M31468 | 0.222 | 1.31 | U41766 | 0.249 | 1.60 |
| L13738 | 0.178 | 1.21 | X79204 | 0.224 | 1.15 | M60459 | 0.250 | 3.17 |
| M29066 | 0.178 | 3.39 | X04076 | 0.225 | 1.13 | X52022 | 0.251 | 2.36 |
| M22491 | 0.179 | 2.30 | Y07684 | 0.226 | 1.43 | AF016268 | 0.251 | 2.05 |
| A06925 | 0.181 | 2.67 | Y00839 | 0.228 | 1.54 | X06745 | 0.254 | 2.10 |
| M92381 | 0.182 | 1.10 | D45021 | 0.229 | 1.08 | J00269+ L42592+ L42601+ L42610+ L42611+ L42612 | 0.255 | 2.71 |
| U02081 | 0.183 | 1.76 | X55885 | 0.229 | 1.12 | L29220 | 0.255 | 2.62 |
| M73482 | 0.183 | 1.44 | M92381 | 0.230 | 1.10 | M95712 | 0.258 | 1.31 |
| U43142 | 0.184 | 3.19 | Z23115; L20121; L20122 | 0.234 | 1.31 | AF017986 | 0.259 | 2.62 |
| D17517 | 0.186 | 1.46 | Z13009 | 0.234 | 6.35 | X77722 | 0.260 | 1.16 |
| M15395 | 0.186 | 2.66 | L06148 | 0.238 | 1.09 | U16296 | 0.260 | 1.77 |
| L41690 | 0.190 | 70.4 | U39412 | 0.239 | 1.08 | L04947; X61656 | 0.261 | 2.94 |
| M30640 | 0.191 | 3.85 | M74715 | 0.239 | 1.44 | U57059 | 0.262 | 2.14 |
| M87339 | 0.192 | 1.42 | M28210 | 0.239 | 1.12 | U47686 | 0.262 | 1.35 |
| D10924 | 0.194 | 2.71 | L09260 | 0.241 | 1.11 | U84401 | 0.262 | 1.94 |
| X51521 | 0.196 | 1.14 | U79299 | 0.246 | 1.15 | U14971 | 0.263 | 1.59 |
| D13316 | 0.196 | 1.73 | U10061 | 0.246 | 1.44 | D26512; X83535 | 0.263 | 1.76 |
| U10421 | 0.196 | 1.22 | D23672 | 0.249 | 1.18 | M60974 | 0.264 | 1.38 |
| Z26317; S64273 | 0.197 | 2.24 | S67368 | 0.249 | 1.47 | X05360 | 0.264 | 1.73 |
| Z30094 | 0.197 | 1.20 | D12676 | 0.249 | 1.08 | U15642 | 0.267 | 1.53 |
| M37981 | 0.199 | 2.18 | X54297 | 0.250 | 1.14 | X56134; M14144 | 0.267 | 1.57 |
| X04602; M14584 | 0.200 | 1.89 | K01911 | 0.253 | 1.16 | U46461 | 0.271 | 1.22 |
| M29142 | 0.200 | 2.42 | M15856 | 0.254 | 1.39 | J05633 | 0.271 | 1.72 |
| U15306 | 0.201 | 1.43 | L19713 | 0.255 | 1.21 | U16306; X15998; U26555; D32039 | 0.272 | 1.71 |
| U08015 | 0.202 | 2.94 | M11058 | 0.256 | 1.14 | M74088; M73548 | 0.274 | 1.05 |
| J02703; M25108 | 0.203 | 2.44 | L08424 | 0.256 | 1.25 | M14219 | 0.275 | 1.61 |
| L14837 | 0.203 | 1.26 | M31659 | 0.258 | 1.33 | L15344 | 0.277 | 2.36 |
| M32315+ M55994 | 0.203 | 216 | M89473 | 0.258 | 94.1 | L38518 | 0.278 | 5.15 |
| M86492 | 0.204 | 1.21 | Z15108 | 0.264 | 1.44 | U24153 | 0.280 | 1.53 |
| X54936 | 0.206 | 1.45 | D30648 | 0.264 | 1.15 | M76125 | 0.284 | 1.51 |
| X69391 | 0.206 | 1.16 | U60520; U58143; X98172; X98173; X98174; AF00962 | 0.269 | 1.43 | X57527 | 0.284 | 2.22 |
| J03358 | 0.207 | 5.36 | D26309 | 0.270 | 1.20 | U32169 | 0.289 | 3.13 |
| M97287 | 0.207 | 1.14 | M29273 | 0.271 | 1.17 | M81933 | 0.291 | 2.10 |
| L36719 | 0.209 | 3.01 | U24105 | 0.274 | 1.22 | U78798; L81153 | 0.291 | 1.27 |
| J04040 | 0.210 | 2.13 | M77844 | 0.279 | 1.11 | X02811; X02744; M12783; M16288 | 0.292 | 1.30 |
| M28214 | 0.211 | 3.85 | S75313 | 0.279 | 1.10 | U34819+ U07620 | 0.292 | 1.25 |
| M31213+ M57464 | 0.211 | 1.64 | U31906 | 0.280 | 1.19 | X60188 | 0.292 | 1.17 |
| U09578 | 0.211 | 2.21 | S69200 | 0.283 | 1.36 | J05556 | 0.293 | 2.27 |
| X69111 | 0.212 | 5.10 | L31951 | 0.284 | 1.18 | M96577 | 0.293 | 1.59 |
| L19185+ Z22548; X82321 | 0.214 | 1.12 | M22199 | 0.287 | 1.18 | M96955+ M96956 | 0.295 | 1.14 |
| M19720 | 0.214 | 2.89 | X81411 | 0.287 | 293 | X63629 | 0.295 | 1.55 |
| X15219 | 0.217 | 1.23 | L11695 | 0.288 | 2.05 | M62403 | 0.296 | 2.09 |
| M12807 | 0.217 | 1.77 | S70609 | 0.289 | 1.54 | J04599 | 0.296 | 2.14 |
| X79929 | 0.218 | 3.80 | D00510 | 0.292 | 1.12 | M77349 | 0.296 | 1.99 |
| L07594 | 0.220 | 1.34 | L06132 | 0.292 | 1.09 | M73812 | 0.297 | 1.78 |
| M87338 | 0.221 | 2.04 | U77088 | 0.294 | 1.18 | AF035752; U32114 | 0.299 | 1.85 |
| M63896 | 0.224 | 1.45 | U59877 | 0.294 | 1.24 | X84740 | 0.300 | 19.4 |
| U01839 | 0.226 | 1.64 | L34075 | 0.295 | 1.18 | X53587; X52186; X51841 | 0.303 | 1.31 |
| J04145 | 0.226 | 1.67 | U12541 | 0.296 | 1.33 | U09579; L25610 | 0.304 | 1.58 |
| M74524 | 0.226 | 1.18 | U06233 | 0.301 | 1.44 | U82532 | 0.311 | 2.27 |
| X08058; M24485 | 0.228 | 1.57 | X59727 | 0.302 | 1.16 | Z12020; M92424 | 0.313 | 7.62 |
| L76224 | 0.232 | 1.77 | Y09567 | 0.304 | 1.13 | U04441 | 0.314 | 1.89 |
| U30473 | 0.232 | 2.44 | U59747 | 0.306 | 1.08 | X72755 | 0.319 | 1.59 |
| U11791; U12685 | 0.236 | 1.19 | S45630 | 0.306 | 1.49 | L31951 | 0.320 | 1.40 |
| L27211 | 0.239 | 1.61 | M86400 | 0.307 | 1.12 | M13228 | 0.322 | 1.92 |
| M27492 | 0.245 | 1.82 | L41939 | 0.309 | 1.83 | X52541; M62829 | 0.323 | 1.31 |
| M58603 | 0.248 | 1.98 | L37792 | 0.309 | 1.13 | J03250 | 0.325 | 1.67 |
| U59747 | 0.248 | 1.76 | X54131 | 0.311 | 1.27 | M34570 | 0.325 | 1.77 |
| M83221 | 0.249 | 2.30 | L34057; L33477 | 0.312 | 1.40 | M14694; M14695 | 0.325 | 1.87 |
| D10925 | 0.249 | 2.10 | X82260 | 0.313 | 1.18 | U82169 | 0.326 | 1.81 |
| U03187 | 0.250 | 1.96 | L32961 | 0.314 | 1.10 | D49493 | 0.326 | 1.46 |
| L08424 | 0.252 | 1.71 | U39196 | 0.316 | 1.58 | X07820; M30461 | 0.327 | 1.78 |
| J03133 | 0.252 | 1.69 | X62535 | 0.316 | 1.24 | U95299 | 0.330 | 2.43 |
| M22488+ U50330 | 0.253 | 1.23 | U07695 | 0.317 | 4.40 | U45880; U32974 | 0.333 | 1.71 |
| X55122; X58072 | 0.254 | 3.71 | L07590 | 0.317 | 1.16 | X90392; L40817; U06846 | 0.334 | 99.7 |
| L04947; X61656 | 0.255 | 2.01 | X15376 | 0.318 | 1.26 | U75285 | 0.335 | 3.05 |
| U00672 | 0.255 | 2.86 | Y00109 | 0.320 | 1.21 | U05012 | 0.337 | 1.68 |
| X75208 | 0.261 | 1.99 | M37981 | 0.320 | 6.33 | M92642 | 0.337 | 1.40 |
| V00530 | 0.262 | 1.18 | U13021+ U13022 | 0.323 | 1.10 | D16431 | 0.339 | 1.65 |
| U09579; L25610 | 0.267 | 1.49 | M86868 | 0.324 | 3.48 | L07868 | 0.341 | 1.66 |
| Z12020; M92424 | 0.267 | 17.3 | U12779 | 0.325 | 1.18 | U60800 | 0.342 | 1.59 |
| X75342 | 0.267 | 1.57 | U03985 | 0.326 | 1.18 | U20536+ U20537 | 0.346 | 2.13 |
| D10232 | 0.269 | 1.49 | U05012 | 0.328 | 1.63 | L05624 | 0.346 | 1.14 |
| M57765 | 0.270 | 2.22 | L18983 | 0.329 | 1.10 | X52599 | 0.346 | 3.08 |
| V00568 | 0.274 | 1.46 | Z48924 | 0.330 | 1.14 | U28014+ U28015 | 0.347 | 1.46 |
| X00351 | 0.275 | 1.27 | L36719 | 0.332 | 1.32 | U25994; U50062 | 0.350 | 1.48 |
| X53587; X52186; X51841 | 0.276 | 1.62 | L19063 | 0.335 | 1.35 | L13616 | 0.353 | 1.16 |
| U02368 | 0.277 | 2.32 | S75989 | 0.340 | 1.22 | J05081 | 0.354 | 3.84 |
| U07418 | 0.278 | 1.44 | L36151 | 0.341 | 1.09 | L07414 | 0.356 | 1.48 |
| M86841 | 0.279 | 1.35 | Y09561 | 0.342 | 1.18 | J03143 | 0.357 | 1.25 |
| M84747 | 0.281 | 1.54 | Z31718 | 0.342 | 1.49 | M57627 | 0.361 | 1.31 |
| X16706 | 0.286 | 2.06 | S76965 | 0.344 | 1.08 | M74178 | 0.361 | 1.68 |
| U43188 | 0.286 | 7.83 | U21108 | 0.345 | 1.12 | L49207+ U43522+ U33284 | 0.363 | 1.18 |
| M68867 | 0.287 | 1.60 | L09247 | 0.346 | 1.15 | M35198; J05522 | 0.363 | 4.26 |
| M13228 | 0.289 | 1.78 | U24660 | 0.347 | 1.22 | U37688 | 0.363 | 2.04 |
| M35203 | 0.291 | 1.29 | U27699 | 0.353 | 1.69 | M64595; M29871 | 0.365 | 1.25 |
| L20046; X69978 | 0.296 | 1.19 | L29126 | 0.354 | 1.15 | U59752 | 0.368 | 1.13 |
| L29277 | 0.297 | 2.26 | D49394 | 0.363 | 3.91 | U21092 | 0.370 | 1.26 |
| M34480; J02764 | 0.301 | 1.91 | D13640 | 0.364 | 1.16 | M33294 | 0.372 | 1.78 |
| U71364 | 0.301 | 1.57 | M24194 | 0.365 | 1.10 | D63878 | 0.372 | 1.18 |
| U32659 | 0.302 | 1.82 | U16996 | 0.367 | 1.17 | M86492 | 0.372 | 1.16 |
| M27288 | 0.306 | 1.76 | L05500 | 0.368 | 1.10 | X06182 | 0.374 | 1.47 |
| M19154; M22045; M22046; Y00083 | 0.308 | 1.22 | L03718 | 0.368 | 1.15 | M99063 | 0.376 | 1.89 |
| Y00285; J03528 | 0.309 | 1.45 | J03727 | 0.369 | 1.40 | M34189 | 0.376 | 3.74 |
| L05624 | 0.311 | 1.19 | S64045 | 0.371 | 10.5 | M80629 | 0.378 | 1.19 |
| M92934 | 0.311 | 1.81 | U61849 | 0.371 | 1.11 | J05070; D10051 | 0.379 | 1.37 |
| M81934; S78187 | 0.312 | 1.41 | L34583 | 0.371 | 1.11 | M21389 | 0.381 | 2.41 |
| X54079 | 0.314 | 1.49 | D38583 | 0.375 | 1.20 | L12002; X16983; X15356 | 0.388 | 4.46 |
| U08853 | 0.317 | 1.74 | Y09689 | 0.377 | 1.20 | M25753 | 0.393 | 1.16 |
| M28372 | 0.318 | 1.17 | M23254 | 0.382 | 1.20 | AF017988 | 0.395 | 2.02 |
| X68486 | 0.321 | 11.5 | X07876 | 0.383 | 1.28 | D14012 | 0.396 | 3.10 |
| U15172 | 0.321 | 3.82 | X02761; K00799; K02273; X00307; X00739 | 0.384 | 1.17 | X07282; Y00291 | 0.397 | 1.19 |
| Y00483; M21304 | 0.328 | 98.1 | D14710 | 0.385 | 1.07 | D14838 | 0.399 | 1.59 |
| U04806; U03858 | 0.328 | 90.7 | M31158 | 0.385 | 1.42 | X74295 | 0.400 | 1.79 |
| Z30425 | 0.328 | 12.1 | J04543 | 0.387 | 1.08 | D83542 | 0.401 | 2.48 |
| D21090 | 0.330 | 1.46 | M18391 | 0.387 | 1.24 | U11791; U12685 | 0.403 | 1.22 |
| X87838; Z19054 | 0.331 | 1.22 | X12548 | 0.388 | 1.11 | X51602+ U01134 | 0.404 | 1.21 |
| L33264 | 0.332 | 1.22 | Y00762 | 0.388 | 1.67 | L26318 | 0.405 | 1.17 |
| U04847 | 0.333 | 1.11 | U07158 | 0.392 | 1.08 | M13982 | 0.408 | 1.95 |
| M28212 | 0.334 | 1.20 | L15388 | 0.394 | 1.14 | X66360 | 0.409 | 1.66 |
| M64673 | 0.336 | 1.16 | M20560; J03899 | 0.394 | 1.25 | U33920 | 0.410 | 2.39 |
| L12002; X16983; X15356 | 0.337 | 1.70 | M36340 | 0.395 | 1.14 | L37882 | 0.410 | 2.49 |
| J03634 | 0.338 | 1.63 | M65105 | 0.399 | 2.38 | M73980 | 0.411 | 2.15 |
| X06182 | 0.340 | 1.49 | M93718 | 0.400 | 1.44 | D21337; U04845 | 0.412 | 1.97 |
| U60520; U58143; X98172; AF00962 | 0.345 | 25.7 | X12454 | 0.404 | 1.23 | U18321+ X83544 | 0.412 | 1.27 |
| L05515 | 0.347 | 1.74 | J04173 | 0.407 | 1.05 | X69550 | 0.415 | 1.09 |
| U14187 | 0.352 | 1.92 | X97198 | 0.407 | 1.32 | M21626 | 0.417 | 2.17 |
| X51688 | 0.352 | 1.63 | M95167 | 0.409 | 1.86 | X68742 | 0.425 | 1.77 |
| D38305 | 0.358 | 1.12 | D10995 | 0.410 | 2.30 | X02492 | 0.427 | 1.43 |
| M25753 | 0.358 | 1.13 | X15187; M33716 | 0.411 | 1.09 | M87339 | 0.431 | 1.36 |
| U07616 | 0.358 | 2.33 | M15169 | 0.414 | 3.86 | M15400 | 0.433 | 1.11 |
| M93426 | 0.361 | 1.09 | U34819+ U07620 | 0.416 | 1.07 | U82938 | 0.433 | 1.18 |
| M73238 | 0.365 | 1.14 | Z25470 | 0.419 | 5.84 | X56807 | 0.436 | 1.61 |
| M29645 | 0.365 | 1.67 | U18728; U21128 | 0.420 | 1.22 | L34060 | 0.443 | 1.20 |
| U05875 | 0.365 | 1.19 | X12453 | 0.421 | 1.20 | U13737 | 0.445 | 1.22 |
| X92669 | 0.366 | 1.19 | X85030 | 0.424 | 1.67 | U22398 | 0.445 | 1.58 |
| M28882 | 0.367 | 1.34 | X12656 | 0.424 | 1.06 | L12350 | 0.446 | 2.97 |
| U45880; U32974 | 0.368 | 1.71 | X78669 | 0.427 | 1.08 | L34056 | 0.447 | 2.00 |
| D26156 | 0.370 | 1.27 | L08603 | 0.428 | 1.20 | D31784 | 0.450 | 1.46 |
| M97676 | 0.371 | 1.33 | U10886 | 0.430 | 1.09 | J00124 | 0.453 | 1.70 |
| U57456 | 0.375 | 1.12 | M23379 | 0.433 | 1.11 | U78876 | 0.455 | 1.42 |
| D38122; U08137 | 0.375 | 2.36 | X65964 | 0.433 | 1.60 | U69611 | 0.457 | 1.14 |
| M54915 | 0.390 | 1.75 | V00530 | 0.435 | 1.13 | U12597 | 0.458 | 1.78 |
| U01160 | 0.391 | 2.20 | L14778 | 0.438 | 1.08 | L40636 | 0.459 | 2.86 |
| X02812; J05114 | 0.392 | 1.11 | M61916 | 0.439 | 1.19 | L36034 | 0.461 | 1.49 |
| Y00787 | 0.393 | 1.71 | D30037 | 0.440 | 1.13 | U11690 | 0.463 | 1.92 |
| L16785+ M36981 | 0.393 | 1.09 | L40157 | 0.440 | 1.08 | L05148 | 0.463 | 1.84 |
| M31630 | 0.393 | 1.90 | M34668 | 0.442 | 1.11 | M92993; X80031 | 0.466 | 2.26 |
| J04156 | 0.393 | 1.70 | M29551 | 0.447 | 1.08 | D89667 | 0.466 | 1.13 |
| X65778+ X51943+ M13361 | 0.393 | 1.53 | L05147 | 0.449 | 1.13 | X80692 | 0.467 | 1.24 |
| U03882 | 0.394 | 1.28 | M11233 | 0.449 | 1.06 | X66357 | 0.467 | 1.73 |
| L04282 | 0.395 | 1.14 | M13077 | 0.450 | 1.17 | U63295 | 0.474 | 1.81 |
| M62505 | 0.398 | 1.57 | U60519 | 0.456 | 1.22 | D30751+ M22490 | 0.476 | 2.03 |
| D11117 | 0.398 | 1.65 | L76224 | 0.456 | 1.96 | M20566; X12830 | 0.476 | 2.15 |
| M62831 | 0.401 | 1.28 | Z11933 | 0.456 | 1.28 | D13804 | 0.477 | 4.17 |
| X02751 | 0.406 | 1.43 | L12387 | 0.461 | 1.12 | M23410; Z68228 | 0.477 | 1.37 |
| P09488; X68676; S01719 | 0.407 | 1.24 | X70697 | 0.461 | 2.65 | L25851 | 0.481 | 1.27 |
| M59040 | 0.407 | 1.41 | D63475 | 0.464 | 1.13 | M59911 | 0.483 | 1.47 |
| A09781 | 0.408 | 1.77 | U57092 | 0.467 | 1.29 | U68162 | 0.484 | 1.49 |
| X12794 | 0.409 | 1.16 | M63960 | 0.469 | 1.10 | M36089 | 0.484 | 1.39 |
| L19606 | 0.411 | 1.55 | Z26634 | 0.469 | 1.17 | M59964 | 0.484 | 1.25 |
| L07541 | 0.412 | 1.31 | U19252 | 0.474 | 1.09 | L42592; L00205 | 0.485 | 1.72 |
| X53655; M37763 | 0.412 | 1.71 | U16306; X15998; U26555; D32039 | 0.477 | 1.09 | D78367 | 0.487 | 1.60 |
| X79389 | 0.413 | 1.19 | D17516 | 0.477 | 1.11 | L11373 | 0.492 | 1.14 |
| M15024 | 0.416 | 1.39 | U92436 | 0.479 | 1.11 | L06139 | 0.493 | 1.64 |
| U10117 | 0.420 | 1.11 | X53143 | 0.487 | 1.09 | X68203; X69878; U43143 | 0.494 | 1.43 |
| U32944 | 0.421 | 1.08 | M88461; M84755 | 0.491 | 1.24 | U18671; M97934 | 0.496 | 1.29 |
| M14200 | 0.422 | 1.11 | Z35309 | 0.495 | 1.25 | U45879+ U37547 | 0.496 | 1.10 |
| D49394 | 0.423 | 1.52 | U50352 | 0.495 | 1.87 | M21574 | 0.497 | 1.16 |
| U63139 | 0.425 | 1.21 | M64676 | 0.495 | 1.20 | M65062 | 0.499 | 1.59 |
| M62424 | 0.426 | 2.12 | X77307 | 0.496 | 2.02 | X66945; M34641; M34186; M37722+ M63887+ M63888+ M63889 | 0.500 | 1.11 |
| M23452 | 0.427 | 2.06 | Z33905 | 0.496 | 1.75 | A26792 | 0.502 | 1.45 |
| L07414 | 0.428 | 2.11 | J03202 | 0.498 | 1.26 | M77198; M95936 | 0.504 | 1.34 |
| M36711 | 0.431 | 2.36 | U22456 | 0.500 | 1.12 | M15476; D00244 | 0.508 | 1.46 |
| J03143 | 0.431 | 1.20 | M34667 | 0.502 | 1.08 | X66364 | 0.508 | 1.13 |
| X75042 | 0.432 | 1.32 | U78876 | 0.507 | 1.24 | X85978 | 0.509 | 1.91 |
| M81695; Y00093 | 0.434 | 1.55 | Y00757 | 0.510 | 1.05 | M31470 | 0.510 | 1.14 |
| M63928 | 0.435 | 1.39 | Z67743 | 0.511 | 1.29 | U41745 | 0.510 | 1.09 |
| X76132 | 0.437 | 1.51 | M84489 | 0.511 | 1.09 | U08839; M83246; X51675 | 0.511 | 1.56 |
| A14844 | 0.438 | 1.63 | X63282; Z50053 | 0.511 | 1.40 | X02812; J05114 | 0.512 | 1.07 |
| L14754 | 0.439 | 1.49 | U07364 | 0.512 | 1.36 | Y00815+ X69398 | 0.513 | 1.20 |
| U59863+ U63830 | 0.442 | 1.09 | M86757 | 0.512 | 1.33 | M29870; M31467 | 0.513 | 1.10 |
| M31158 | 0.443 | 1.25 | M95678 | 0.513 | 1.08 | M65199 | 0.513 | 1.61 |
| L20815 | 0.446 | 1.72 | X54469; M28019 | 0.515 | 1.24 | U38276 | 0.514 | 2.27 |
| M27544+ M37484 | 0.446 | 1.42 | X77533 | 0.520 | 1.22 | U72661 | 0.514 | 1.10 |
| U45879+ U37547 | 0.448 | 1.08 | J02611 | 0.523 | 1.10 | X66365 | 0.514 | 1.66 |
| U60800 | 0.451 | 1.29 | Y00264 | 0.525 | 1.19 | M99061; S43646 | 0.519 | 1.58 |
| U18321+ X83544 | 0.454 | 1.38 | X65362+ X65361; M94055+ X82835 | 0.526 | 1.18 | M63488 | 0.521 | 1.14 |
| M62302 | 0.457 | 1.51 | S62045 | 0.527 | 1.13 | M24857; M38258; M57707; M32074 | 0.521 | 1.27 |
| D10495 | 0.459 | 1.31 | M28214 | 0.528 | 1.31 | J03202 | 0.522 | 1.55 |
| M13150 | 0.466 | 1.37 | M35533 | 0.528 | 1.41 | X70904; X91171 | 0.522 | 1.24 |
| X74295 | 0.468 | 1.13 | L07594 | 0.538 | 1.28 | X52946+ D90226+ M57399 | 0.525 | 1.05 |
| X06256 | 0.473 | 1.39 | X93499 | 0.542 | 1.07 | L23808 | 0.526 | 1.69 |
| M75914 | 0.473 | 1.15 | M35531 | 0.543 | 1.08 | M60316 | 0.526 | 1.89 |
| X60957; S89716 | 0.474 | 1.50 | X74142; X78202 | 0.545 | 1.06 | D13365; M93311 | 0.526 | 1.09 |
| U03056 | 0.475 | 1.76 | X75756 | 0.547 | 1.24 | L36052; L36051; U11025 | 0.528 | 1.35 |
| M34356 | 0.476 | 1.48 | X04106 | 0.547 | 1.06 | M58051+ X58255 | 0.532 | 1.81 |
| X76648 | 0.478 | 1.19 | M31630 | 0.549 | 1.07 | U69276 | 0.533 | 1.23 |
| L25259 | 0.479 | 2.18 | M36430 | 0.549 | 1.13 | U63139 | 0.534 | 1.73 |
| M31159; M35878 | 0.479 | 1.18 | L18964 | 0.550 | 1.07 | L24564 | 0.541 | 2.79 |
| J03746; B28083 | 0.481 | 1.12 | X95425 | 0.550 | 1.49 | M99437 | 0.545 | 1.48 |
| M60718 | 0.485 | 1.51 | X71661 | 0.552 | 1.16 | U14407 | 0.546 | 1.27 |
| U09607 | 0.490 | 1.29 | U31215 | 0.552 | 1.48 | U00001 | 0.547 | 1.11 |
| U56390; U60521 | 0.491 | 1.56 | U37673 | 0.558 | 1.13 | L17075 | 0.547 | 1.39 |
| M83941 | 0.496 | 1.59 | D10326 | 0.561 | 1.14 | U13021+ U13022 | 0.549 | 1.28 |
| M36717 | 0.497 | 1.23 | U63533 | 0.563 | 1.38 | M60314 | 0.555 | 1.71 |
| M96824 | 0.498 | 1.16 | U03504 | 0.565 | 1.13 | U28811; U64791 | 0.559 | 1.17 |
| K03214; X03996 | 0.500 | 1.26 | U45955 | 0.569 | 1.20 | U35113 | 0.560 | 1.40 |
| D10202 | 0.501 | 1.40 | L06139 | 0.571 | 1.16 | D85815 | 0.562 | 1.30 |
| U03905 | 0.504 | 1.33 | X93920 | 0.572 | 1.21 | U09304 | 0.563 | 1.46 |
| X67951 | 0.505 | 1.25 | L23958 | 0.572 | 1.09 | D21235 | 0.567 | 1.99 |
| M20132; J03180 | 0.508 | 1.32 | L26232 | 0.573 | 1.06 | X05199 | 0.569 | 1.46 |
| M61176 | 0.509 | 1.61 | U41901 | 0.574 | 1.06 | X03212 | 0.570 | 1.77 |
| U02683 | 0.510 | 1.49 | X16841; S71824 | 0.574 | 1.07 | L26081 | 0.571 | 1.35 |
| M19722 | 0.511 | 1.41 | M34064; X57548; X54315; S42303 | 0.575 | 1.04 | L29222 | 0.574 | 1.21 |
| M36542 | 0.511 | 1.95 | D13988 | 0.575 | 1.10 | V00530 | 0.576 | 1.07 |
| M95489 | 0.511 | 1.30 | X72304 | 0.577 | 1.32 | L03840 | 0.577 | 2.50 |
| X59738 | 0.512 | 1.09 | M12529 | 0.580 | 1.09 | D49742; S83182 | 0.578 | 1.33 |
| M65199 | 0.514 | 1.34 | U09304 | 0.582 | 1.24 | X04571 | 0.578 | 1.43 |
| M81601 | 0.520 | 1.13 | M97759 | 0.583 | 1.15 | Y11416 | 0.579 | 1.54 |
| U60519 | 0.520 | 1.06 | U66702 | 0.585 | 1.09 | M22488+ U50330 | 0.580 | 1.17 |
| M34664 | 0.521 | 1.18 | S81944 | 0.585 | 1.23 | X76132 | 0.581 | 1.18 |
| M23197 | 0.525 | 1.48 | X68274 | 0.585 | 1.10 | X04688; J03478 | 0.581 | 1.41 |
| L00587 | 0.527 | 1.30 | X56667 | 0.586 | 1.06 | U12134 | 0.588 | 1.52 |
| L22474 | 0.529 | 1.50 | U56976 | 0.590 | 1.15 | X87838; Z19054 | 0.590 | 1.09 |
| L12579 | 0.531 | 1.31 | L08807 | 0.593 | 1.04 | X97057 | 0.598 | 1.30 |
| L13740 | 0.536 | 1.34 | X74210 | 0.593 | 1.07 | M84820; X63522; S54072 | 0.600 | 1.14 |
| X16841; S71824 | 0.536 | 1.13 | K03020 | 0.594 | 1.06 | X03168 | 0.601 | 1.23 |
| M59964 | 0.538 | 1.28 | L07807 | 0.598 | 1.68 | M73780 | 0.603 | 1.18 |
| M18391 | 0.539 | 1.14 | AF007548 | 0.598 | 1.06 | X02761; K00799; K02273; X00307; X00739 | 0.606 | 1.28 |
| M74178 | 0.539 | 1.14 | D26135 | 0.600 | 1.11 | Z24680 | 0.607 | 1.18 |
| M67454 | 0.551 | 2.54 | U12535 | 0.601 | 1.23 | M77830; J05211 | 0.609 | 1.47 |
| X53799 | 0.551 | 1.32 | U11700 | 0.601 | 1.07 | L06801 | 0.614 | 1.27 |
| X72755 | 0.551 | 1.53 | U25138 | 0.604 | 1.26 | U26403 | 0.617 | 1.14 |
| X80692 | 0.554 | 1.29 | X56741 | 0.610 | 1.14 | J03040 | 0.622 | 1.19 |
| D26309 | 0.555 | 1.08 | X92396 | 0.613 | 1.49 | M57765 | 0.626 | 1.28 |
| D26155 | 0.555 | 1.07 | M55268 | 0.614 | 1.04 | X95456 | 0.626 | 1.21 |
| U14971 | 0.558 | 1.09 | M30185 | 0.614 | 1.09 | S66431 | 0.626 | 1.38 |
| D90209 | 0.558 | 1.13 | L13943 | 0.615 | 1.07 | AF028593 | 0.635 | 1.12 |
| M13981 | 0.565 | 1.34 | L13616 | 0.616 | 1.05 | K00558 | 0.636 | 1.16 |
| D13889 | 0.566 | 1.18 | A03911 | 0.631 | 1.07 | X14420 | 0.638 | 1.29 |
| L20422 | 0.566 | 1.09 | X53655; M37763 | 0.632 | 1.17 | M18082; J02685 | 0.643 | 1.53 |
| M92287 | 0.569 | 1.42 | U66406 | 0.633 | 1.15 | M54968 | 0.643 | 1.08 |
| L06623 | 0.577 | 1.12 | L06147 | 0.634 | 1.06 | U29343 | 0.644 | 2.02 |
| X59798; M64349 | 0.579 | 1.25 | M32977; M27281 | 0.639 | 1.12 | X72925; Z34522 | 0.646 | 1.49 |
| M35198; J05522 | 0.583 | 1.33 | X04366 | 0.639 | 1.16 | X14454 | 0.648 | 1.19 |
| M32865; S38729 | 0.583 | 1.27 | M94172 | 0.639 | 1.10 | U43901 | 0.649 | 1.12 |
| U28014+ U28015 | 0.586 | 1.22 | J04182 | 0.642 | 1.04 | M37825 | 0.650 | 1.57 |
| X58022 | 0.592 | 1.19 | X70476 | 0.650 | 1.03 | U77845 | 0.652 | 1.23 |
| U25994; U50062 | 0.604 | 1.27 | U39905 | 0.653 | 1.13 | L41939 | 0.653 | 1.38 |
| J05633 | 0.609 | 1.41 | X57206 | 0.656 | 1.04 | J05593 | 0.654 | 1.22 |
| X04434; M24599 | 0.610 | 1.99 | M92302+ M92303+ L06112 | 0.656 | 1.18 | L07540 | 0.660 | 1.61 |
| L34587 | 0.617 | 1.05 | X77130 | 0.656 | 1.39 | M29971 | 0.661 | 1.34 |
| U02687 | 0.618 | 1.32 | J02902 | 0.656 | 1.02 | M30704 | 0.662 | 1.28 |
| M30704 | 0.622 | 1.28 | J03778 | 0.658 | 1.03 | S83171; Z35491 | 0.662 | 1.03 |
| L13616 | 0.623 | 1.13 | J05252 | 0.659 | 1.05 | M98776 | 0.664 | 1.43 |
| D28118 | 0.626 | 1.07 | M81830 | 0.665 | 1.12 | M99487 | 0.664 | 1.23 |
| X55504 | 0.626 | 1.26 | M62397 | 0.671 | 1.10 | M24795 | 0.666 | 1.21 |
| U12134 | 0.627 | 1.18 | U30461 | 0.673 | 1.07 | U43527 | 0.667 | 1.25 |
| U43746 | 0.632 | 1.31 | U25341 | 0.685 | 1.80 | X66358 | 0.669 | 1.33 |
| M60828 | 0.634 | 1.40 | X94552 | 0.686 | 1.23 | U66197 | 0.670 | 1.08 |
| L14922 | 0.634 | 1.14 | U32944 | 0.693 | 1.05 | K00650 | 0.670 | 1.18 |
| J03241 | 0.641 | 1.19 | L09210 | 0.696 | 1.14 | X54936 | 0.672 | 1.26 |
| S40706; S62138 | 0.642 | 1.20 | U07616 | 0.696 | 1.14 | M15990 | 0.674 | 1.18 |
| U12140 | 0.642 | 1.07 | U09579; L25610 | 0.698 | 1.14 | U33286 | 0.676 | 1.12 |
| U43408 | 0.643 | 1.96 | M27507 | 0.700 | 1.04 | U77949 | 0.676 | 1.29 |
| L35233 | 0.646 | 1.32 | M72393 | 0.704 | 1.11 | M32865; S38729 | 0.678 | 1.17 |
| M11717 | 0.648 | 1.24 | U38545 | 0.704 | 1.10 | M14505 | 0.680 | 1.25 |
| J04536 | 0.649 | 1.25 | X97074 | 0.708 | 1.10 | U32659 | 0.684 | 1.30 |
| U34819+ U07620 | 0.650 | 1.07 | M64572 | 0.709 | 1.05 | X02530 | 0.687 | 1.15 |
| M74816 | 0.655 | 1.04 | U40370 | 0.719 | 1.16 | X56654 | 0.688 | 1.17 |
| M97190 | 0.656 | 1.15 | M65212 | 0.719 | 1.08 | M92934 | 0.695 | 1.37 |
| X72304 | 0.661 | 1.26 | X72964 | 0.726 | 1.04 | U63131 | 0.702 | 1.06 |
| M76673 | 0.668 | 1.49 | S62907 | 0.727 | 1.05 | U56390; U60521 | 0.703 | 1.09 |
| L35253; L35263 | 0.669 | 1.07 | D21260 | 0.734 | 1.06 | X63454 | 0.707 | 1.59 |
| M87290 | 0.669 | 1.21 | M57414 | 0.734 | 1.18 | M77227 | 0.719 | 1.24 |
| L06139 | 0.670 | 1.13 | L25124; D28472 | 0.738 | 1.59 | X05610 | 0.727 | 1.31 |
| U47414; L49506 | 0.671 | 1.20 | U40215 | 0.739 | 1.06 | U24152 | 0.727 | 1.09 |
| M35410 | 0.671 | 1.07 | X78565; M55618 | 0.739 | 1.10 | U16752; L36033 | 0.727 | 1.19 |
| S90469 | 0.671 | 1.36 | M62843 | 0.741 | 1.05 | M80634+ U11814+ X52832; M35718+ M87771+ M87772 | 0.729 | 1.36 |
| U13897 | 0.674 | 1.06 | J04569 | 0.746 | 1.05 | AF010309 | 0.729 | 1.19 |
| M31523 | 0.675 | 1.16 | D21243; S34389 | 0.747 | 1.05 | X15879 | 0.733 | 1.27 |
| M14764 | 0.675 | 1.13 | M61829 | 0.747 | 1.04 | AF022385 | 0.739 | 1.05 |
| D28468 | 0.676 | 1.16 | U13699; M87507; X65019 | 0.748 | 1.08 | AF010127; Y14039; Y14040 | 0.741 | 1.17 |
| L32976 | 0.676 | 1.05 | X13227 | 0.748 | 1.13 | M35410 | 0.741 1.08 | |
| M92843 | 0.677 | 1.25 | L11285 | 0.748 | 1.10 | L09753 | 0.744 | 1.17 |
| X07024 | 0.680 | 1.31 | M86841 | 0.754 | 1.11 | M34064; X57548; X54315; S42303 | 0.746 | 1.05 |
| M26062 | 0.684 | 1.20 | X54938 | 0.756 | 1.10 | L29511; M96995 | 0.747 | 1.06 |
| K03222 | 0.684 | 1.49 | M93426 | 0.761 | 1.13 | L25081 | 0.749 | 1.07 |
| X53586; X59512 | 0.686 | 1.18 | X12646 | 0.762 | 1.02 | L07541 | 0.753 | 1.11 |
| L31881 | 0.690 | 1.09 | X79510 | 0.763 | 1.07 | X80343 | 0.754 | 1.05 |
| U10324 | 0.693 | 1.19 | L14865 | 0.763 | 1.35 | U23765; U16811; X84213 | 0.754 | 1.29 |
| M81757 | 0.696 | 1.03 | X14298; M18533; M17154; M18026 | 0.765 | 1.06 | M13194 | 0.755 | 1.15 |
| L24564 | 0.697 | 1.26 | Z26653 | 0.765 | 1.08 | U38545 | 0.756 | 1.17 |
| M11220 | 0.699 | 1.25 | D78579 | 0.769 | 1.04 | X55525; J03464 | 0.761 | 1.22 |
| M36430 | 0.700 | 1.06 | X77748 | 0.769 | 1.31 | U90875 | 0.761 | 1.07 |
| D42108 | 0.703 1.08 | | U13737 | 0.773 | 1.05 | X06374 | 0.763 | 1.07 |
| X01057; X01058; X01402 | 0.705 | 1.08 | X17094 | 0.773 | 1.05 | L23959 | 0.764 | 1.11 |
| M65066 | 0.708 | 1.12 | U26648 | 0.774 | 1.03 | L27211 | 0.765 | 1.19 |
| M20566; X12830 | 0.710 | 1.12 | X63745 | 0.775 | 1.04 | U37448 | 0.765 | 1.14 |
| U40343; U20498 | 0.711 | 1.07 | X14034 | 0.775 | 1.05 | U10087; X58957 | 0.767 | 1.27 |
| U29680; Y09397 | 0.712 | 1.35 | L19711 | 0.776 | 1.04 | U66879 | 0.769 | 1.12 |
| M81933 | 0.713 | 1.30 | J05225 | 0.779 | 1.03 | AF000974 | 0.772 | 1.18 |
| M59818 | 0.714 | 1.18 | D29643 | 0.780 | 1.03 | X78565; M55618 | 0.773 | 1.28 |
| X02530 | 0.716 | 1.25 | L36531 | 0.782 | 1.08 | L20046; X69978 | 0.774 | 1.09 |
| U18422 | 0.721 | 1.18 | M80254 | 0.783 | 1.05 | M97016 | 0.776 | 1.15 |
| M68520 | 0.727 | 1.18 | D28538 | 0.783 | 1.13 | L41690 | 0.776 | 1.07 |
| M84489 | 0.728 | 1.13 | X98093 | 0.784 | 1.03 | X52773 | 0.778 | 1.15 |
| L34583 | 0.732 | 1.09 | U18420 | 0.789 | 1.03 | M60278 | 0.779 | 1.06 |
| M96955+ M96956 | 0.736 | 1.12 | M83738 | 0.790 | 1.02 | AF010314 | 0.779 | 1.08 |
| M21097 | 0.742 | 1.19 | L31409 | 0.792 | 1.07 | L11353; Z22664; X72657; L27133 | 0.779 | 1.13 |
| M64752 | 0.745 | 1.04 | X13255 | 0.794 | 1.05 | L34057; L33477 | 0.780 | 1.19 |
| L26318 | 0.745 | 1.17 | X75500 | 0.796 | 1.07 | U59167 | 0.785 | 1.17 |
| U33841 | 0.745 | 1.13 | U51477 | 0.796 | 1.05 | U35835+ U47077 | 0.786 | 1.15 |
| L08187 | 0.750 | 1.15 | X79882 | 0.799 | 1.06 | U40343; U20498 | 0.787 | 1.04 |
| K00065; X02317 | 0.753 | 1.03 | M81882 | 0.803 | 1.05 | M11313 | 0.789 | 1.09 |
| M86528 | 0.757 | 1.19 | U26403 | 0.810 | 1.08 | M55172 | 0.790 | 1.08 |
| M29696 | 0.763 | 1.07 | Z18948 | 0.813 | 1.07 | L11370 | 0.792 | 1.12 |
| U68162 | 0.767 | 1.28 | J00123 | 0.815 | 1.05 | L25676 | 0.798 | 1.10 |
| M14694; M14695 | 0.768 | 1.42 | L20433 | 0.817 | 1.04 | U07707; Z29064 | 0.799 | 1.07 |
| X02811; X02744; M12783; M16288 | 0.768 | 1.19 | U16126 | 0.822 | 1.04 | U40282 | 0.800 | 1.12 |
| X07979 | 0.771 | 1.04 | X95808 | 0.824 | 1.02 | U39657 | 0.806 | 1.11 |
| M74782 | 0.774 | 1.14 | U33267 | 0.824 | 1.06 | U76638 | 0.807 | 1.19 |
| X07282; Y00291 | 0.778 | 1.06 | X64810 | 0.825 | 1.04 | M57730; M37476 | 0.811 | 1.16 |
| X07270 | 0.784 | 1.11 | X52009 | 0.825 | 1.19 | U59863+ U63830 | 0.811 | 1.05 |
| M84820; X63522; S54072 | 0.785 | 1.12 | U00803 | 0.829 | 1.08 | U04806; U03858 | 0.811 | 1.25 |
| M36089 | 0.787 | 1.27 | U70064 | 0.830 | 1.04 | U43195 | 0.814 | 1.05 |
| U07819 | 0.799 | 1.05 | X06661 | 0.832 | 1.03 | L20320 | 0.815 | 1.11 |
| M15400 | 0.801 | 1.04 | J04111 | 0.833 | 1.02 | L29216 | 0.817 | 1.10 |
| U12535 | 0.804 | 1.16 | D31897 | 0.837 | 1.01 | M63167 | 0.818 | 1.12 |
| X59770 | 0.804 | 1.10 | L20688 | 0.838 | 1.09 | J02703; M25108 | 0.822 | 1.07 |
| X06745 | 0.805 | 1.07 | M14221 | 0.841 | 1.02 | U47413 | 0.824 | 1.09 |
| U45878+ U37546 | 0.808 | 1.19 | L34774 | 0.842 | 1.04 | M74091 | 0.824 | 1.07 |
| U47413 | 0.809 | 1.14 | L26318 | 0.843 | 1.05 | J03210; J05471 | 0.827 | 1.18 |
| U10323 | 0.813 | 1.07 | X82103 | 0.849 | 1.06 | K02770 | 0.833 | 1.02 |
| X14454 | 0.818 | 1.14 | X05908; M19383; J03264 | 0.850 | 1.04 | X86779 | 0.837 | 1.05 |
| U40282 | 0.820 | 1.23 | X76981 | 0.852 | 1.13 | X14445 | 0.839 | 1.15 |
| M14648; J02826; M18365 | 0.822 | 1.07 | Z21876 | 0.852 | 1.08 | M26880 | 0.840 | 1.06 |
| X03438 | 0.823 | 1.18 | U33551 | 0.853 | 1.04 | U76456 | 0.842 | 1.13 |
| X15218 | 0.823 | 1.07 | X80343 | 0.856 | 1.02 | M85289 | 0.843 | 1.27 |
| Z29090 | 0.827 | 1.05 | D64053 | 0.858 | 1.03 | X51521 | 0.844 | 1.05 |
| X17648 | 0.827 | 1.11 | X80692 | 0.860 | 1.04 | M32315+ M55994 | 0.847 | 1.09 |
| M15169 | 0.830 | 1.17 | D89858 | 0.860 | 1.06 | S57153+ S57160 | 0.847 | 1.02 |
| D15050 | 0.831 | 1.05 | U18840 | 0.861 | 1.02 | L16785+ M36981 | 0.851 | 1.02 |
| M36429 | 0.835 | 1.15 | J04046 | 0.866 | 1.01 | M74387 | 0.851 | 1.04 |
| U13021+ U13022 | 0.835 | 1.09 | U38654 | 0.875 | 1.07 | U60519 | 0.858 | 1.03 |
| M91196 | 0.836 | 1.11 | S56143 | 0.881 | 1.02 | X52426; X07696; X62571 | 0.860 | 1.14 |
| L29222 | 0.839 | 1.03 | L35901 | 0.882 | 1.18 | A09781 | 0.862 | 1.08 |
| U09564 | 0.843 | 1.04 | AF016917 | 0.883 | 1.02 | U83508 | 0.862 | 1.16 |
| M63488 | 0.845 | 1.03 | M28215 | 0.883 | 1.01 | U43142 | 0.864 | 1.19 |
| M22199 | 0.846 | 1.06 | X58079 | 0.886 | 1.02 | M35543+ M57298 | 0.869 | 1.04 |
| K02770 | 0.849 | 1.02 | L06155 | 0.886 | 1.03 | M15518; X07393; M18182 | 0.874 | 1.05 |
| X78686 | 0.850 | 1.14 | M29870; M31467 | 0.891 | 1.01 | X00351 | 0.875 | 1.05 |
| M97675 | 0.854 | 1.15 | L20321 | 0.893 | 1.02 | U84388 | 0.876 | 1.10 |
| M27545; X06318 | 0.855 | 1.04 | X69117 | 0.894 | 1.02 | M28622 | 0.879 | 1.08 |
| D49547 | 0.857 | 1.10 | M59040 | 0.895 | 1.05 | D38122; U08137 | 0.880 | 1.06 |
| M14752; M14753; M14754 | 0.857 | 1.08 | U11690 | 0.896 | 1.08 | Z26317; S64273 | 0.880 | 1.15 |
| M16552 | 0.858 | 1.13 | X52008 | 0.896 | 1.07 | M65290 | 0.881 | 1.06 |
| X16707 | 0.858 | 1.09 | L38734 | 0.897 | 1.05 | U05875 | 0.886 | 1.02 |
| U35835+ U47077 | 0.864 | 1.03 | X66533 | 0.898 | 1.02 | U53786 | 0.886 | 1.11 |
| X03484 | 0.864 | 1.02 | U07139 | 0.898 | 1.02 | X61587 | 0.886 | 1.06 |
| X76981 | 0.866 | 1.16 | M73704 | 0.899 | 1.01 | X03541 | 0.890 | 1.05 |
| M83234 | 0.866 | 1.02 | X14968 | 0.899 | 1.01 | S72008 | 0.891 | 1.02 |
| M91815; L26584 | 0.873 | 1.03 | U07550 | 0.901 | 1.02 | X83441 | 0.891 | 1.02 |
| U15174 | 0.877 | 1.04 | L25119 | 0.901 | 1.02 | M92287 | 0.901 | 1.04 |
| M28211 | 0.877 | 1.05 | U03270 | 0.902 | 1.02 | X17620 | 0.903 | 1.05 |
| X68203; X69878; U43143 | 0.878 | 1.05 | X81120 | 0.903 | 1.03 | M29039 | 0.903 | 1.10 |
| M30938 | 0.884 | 1.14 | X94703 | 0.903 | 1.08 | X05231 | 0.915 | 1.04 |
| L19058 | 0.888 | 1.06 | D16480 | 0.909 | 1.01 | X60957; S89716 | 0.918 | 1.10 |
| M76541 | 0.892 | 1.02 | L25876 | 0.912 | 1.02 | AF01031 2 | 0.921 | 1.03 |
| M37435 | 0.898 | 1.03 | M34175 | 0.912 | 1.01 | M68516; J02639 | 0.921 | 1.05 |
| X59932 | 0.899 | 1.05 | L12260; L12261+ U02326+ M94165 | 0.922 | 1.03 | Z29074; S69510 | 0.922 | 1.07 |
| X02851 | 0.899 | 1.07 | U57093 | 0.922 | 1.03 | L36531 | 0.923 | 1.06 |
| M76125 | 0.899 | 1.05 | X03541 | 0.922 | 1.04 | L34774 | 0.926 | 1.02 |
| U14755 | 0.902 | 1.11 | J02853 | 0.925 | 1.01 | U48801; U43369 | 0.927 | 1.04 |
| M95809 | 0.903 | 1.05 | J03746; B28083 | 0.926 | 1.02 | U33635+ U40271 | 0.928 | 1.03 |
| U21092 | 0.905 | 1.10 | M63635 | 0.929 | 1.02 | L33264 | 0.931 | 1.04 |
| X52425 | 0.906 | 1.04 | X58531 | 0.929 | 1.03 | X74262 | 0.932 | 1.02 |
| M31899 | 0.908 | 1.02 | X75593 | 0.932 | 1.01 | L07594 | 0.941 | 1.02 |
| U13737 | 0.912 | 1.06 | M76180 | 0.937 | 1.02 | L20861 | 0.942 | 1.05 |
| X77722 | 0.912 | 1.04 | L20469 | 0.938 | 1.03 | U33841 | 0.943 | 1.08 |
| M59911 | 0.914 | 1.04 | X65882 | 0.938 | 1.02 | U10564 | 0.943 | 1.04 |
| D11086 | 0.917 | 1.03 | X80818 | 0.939 | 1.04 | Y08622+ X92521 | 0.944 | 1.05 |
| U05040 | 0.919 | 1.01 | U16129 | 0.939 | 1.01 | J04156 | 0.944 | 1.05 |
| D13804 | 0.922 | 1.08 | S45018 | 0.940 | 1.02 | X03484 | 0.948 | 1.01 |
| M28213 | 0.923 | 1.01 | X17622 | 0.943 | 1.03 | U17075; L36844 | 0.949 | 1.05 |
| U57059 | 0.926 | 1.06 | X89416 | 0.946 | 1.01 | U60520; U58143; X98172; AF00962 | 0.950 | 1.04 |
| U16031 | 0.926 | 1.07 | L05597 | 0.950 | 1.02 | Z48482 | 0.953 | 1.04 |
| M16038 | 0.926 | 1.04 | M55284 | 0.952 | 1.04 | AF010315 | 0.954 | 1.04 |
| L31951 | 0.927 | 1.02 | U45879+U 37547 | 0.953 | 1.01 | X04602; M14584 | 0.955 | 1.01 |
| M76446 | 0.927 | 1.08 | L02750 | 0.955 | 1.01 | X76104 | 0.958 | 1.01 |
| M26880 | 0.928 | 1.01 | Y00285;J 03528 | 0.957 | 1.01 | A14844 | 0.962 | 1.02 |
| L22075 | 0.929 | 1.04 | L36983 | 0.959 | 1.01 | M27544+ M37484 | 0.962 | 1.02 |
| M90813+ D13639 | 0.932 | 1.01 | M92303 | 0.959 | 1.05 | M16591 | 0.962 | 1.04 |
| Z23115; L20121; L20122 | 0.935 | 1.02 | Y00414 | 0.961 | 1.01 | Y00285; J03528 | 0.963 | 1.03 |
| X51630 | 0.942 | 1.04 | U14188 | 0.962 | 1.02 | U45878+ U37546 | 0.963 | 1.03 |
| M74387 | 0.946 | 1.02 | X68203;X 69878;U4 3143 | 0.965 | 1.01 | D13866; D14705; L23805; L22080 | 0.964 | 1.01 |
| M14745 | 0.948 | 1.03 | U34051 | 0.965 | 1.01 | M87338 | 0.966 | 1.02 |
| M74088; M73548 | 0.950 | 1.00 | U39657 | 0.967 | 1.01 | U86759 | 0.966 | 1.03 |
| D12614 | 0.951 | 1.06 | X08004 | 0.968 | 1.00 | U12140 | 0.967 | 1.01 |
| U08191 | 0.952 | 1.01 | M86528+S 41522+S4 1540+S41 541 | 0.968 | 1.01 | J02958 | 0.970 | 1.02 |
| M10051; X02160 | 0.955 | 1.03 | X54134 | 0.970 | 1.01 | M31159; M35878 | 0.971 | 1.02 |
| Y09392+ U75380+ U74611+ U83597 | 0.955 | 1.05 | M68941 | 0.972 | 1.00 | M14764 | 0.972 | 1.01 |
| J03171 | 0.957 | 1.01 | U40371 | 0.974 | 1.02 | Y07923 | 0.973 | 1.01 |
| X60592 | 0.958 | 1.02 | AB001835 | 0.976 | 1.01 | U37139 | 0.974 | 1.01 |
| M21616 | 0.962 | 1.01 | M19383 | 0.979 | 1.01 | Y07604 | 0.974 | 1.01 |
| M28210 | 0.964 | 1.01 | M28213 | 0.981 | 1.00 | X87852 | 0.976 | 1.01 |
| M80627 | 0.967 | 1.00 | U09117 | 0.981 | 1.01 | AF007111 | 0.977 | 1.01 |
| M62397 | 0.977 | 1.01 | D25542 | 0.981 | 1.00 | L19063 | 0.977 | 1.02 |
| L36870 | 0.979 | 1.00 | D13380 | 0.982 | 1.00 | X04429; M14083 | 0.978 | 1.01 |
| M21574 | 0.983 | 1.00 | M14780 | 0.984 | 1.01 | U18422 | 0.984 | 1.01 |
| U14575 | 0.986 | 1.01 | M64752 | 0.986 | 1.00 | M60718 | 0.986 | 1.01 |
| L25876 | 0.988 | 1.01 | X78520 | 0.991 | 1.00 | L34058; U59289; U59288 | 0.990 | 1.01 |
| M68516; J02639 | 0.991 | 1.01 | D16593; D16227 | 0.991 | 1.00 | U34051 | 0.990 | 1.01 |
| X56681 | 0.993 | 1.00 | L14754 | 0.991 | 1.00 | K03222 | 0.991 | 1.01 |
| M62810 | 0.996 | 1.00 | X97966 | 0.992 | 1.00 | U49089 | 0.996 | 1.00 |
| M34064; X57548; X54315; S42303 | 0.997 | 1.00 | Y10659 | 0.995 | 1.00 | U04045; L47583 | 0.996 | 1.00 |
| L09210 | | 1.00 | U14971 | 0.996 | 1.00 | M15530 | 0.996 | 1.00 |
| D14012 | | 1.00 | M14200 | 0.996 | 1.00 | X01677 | 0.999 | 1.00 |

### [Example 1]

In this example, we will explain a diagnostic method for schizophrenia by quantifying the expression amount (protein) of the gene encoding a precursor of a receptor of a brain-derived neurotrophic factor (BDNF)/NT-3 amplification factor (registered at GenBank under the number of U12140, hereinafter referred to as "TrkB").

First, the frontal lobe tissue of the patient was subjected to anatomy and proteins were extracted from the frontal lobe tissue. In the same manner, proteins were extracted from the non-patient group and purified. Twenty *µ*g of each of the extracted proteins was subjected to electrophoresis on SDS polyacrylamide gel and then, electrically transferred to a nylon membrane. Subsequently, a TrkB antigen was detected in each of samples on the membrane by the western blotting method using an anti-TrkB antibody. Quantification was made by an ECL luminescent method using a photosensitive film.

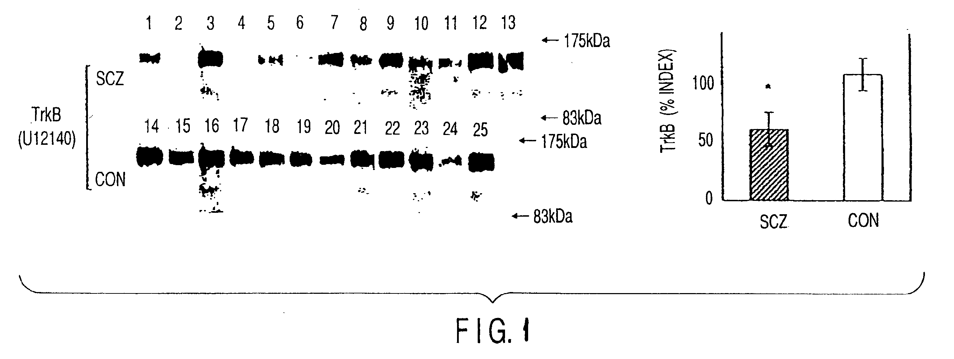

The results are shown in FIG. 1.

Western blots of TrkB of patients are shown in the upper stage (SCZ) and western blots of TrkB of non patients (control) are shown in the lower stage (CON) of FIG. 1.

As is apparent from the graph shown at the right hand side, a relative expression amount of TrkB (protein) is about 110 in the control group and about 60 in the patient group. The expression amount of TrkB of the patient group is significantly low compared to the control group. The patient group presents abnormality.

As shown in the aforementioned example, it is demonstrated that the expression amount of the protein can be used as an index of schizophrenia.

However, the expression amount of the TrkB gene (mRNA) was actually high in schizophrenic patients. Therefore, it should be noted that the expression amount of the protein does not always behave in the same fashion as that of the gene.

Accordingly, when the expression amount of the nucleic acid (gene) is indirectly obtained by quantifying the protein, it is important to know whether the target protein is expressed high or low in the schizophrenic patients, in proportional or inversely proportional to the expression amount of its nucleic acids.

### [Example 2]

In this example, we will explain a diagnostic method for schizophrenia on the basis of the expression amounts of a plurality of genes by using nucleic acids taken from tissues of dead patients and a DNA micro-array.

First, a frontal lobe of a patient's brain was sectioned, and then, RNA was extracted by an acid phenol extraction method and purified. At the same time, pure RNA was prepared from a non-schizophrenic patient as a comparative group. Using the extracted RNA as a template and radio-labeled nucleic acids as substrates (building blocks), cDNA is synthesized with the aid of reverse transcriptase to measure the contents of mRNAs of a plurality of genes in the tissue cells.

Radio-labeled cDNA of each of patient's samples is linked to the nucleic acids on a DNA micro-array (Bland name: Atlas Human cDNA Expression Array, manufactured by Cloneteck). On the DNA micro-array, 588 types of nucleic acids of a human gene are spotted two for each. After non-bound radio-labeled cDNAs are washed away from the DNA micro array, signal intensity of each of the 588 types of human genes was measured and visualized as an image.

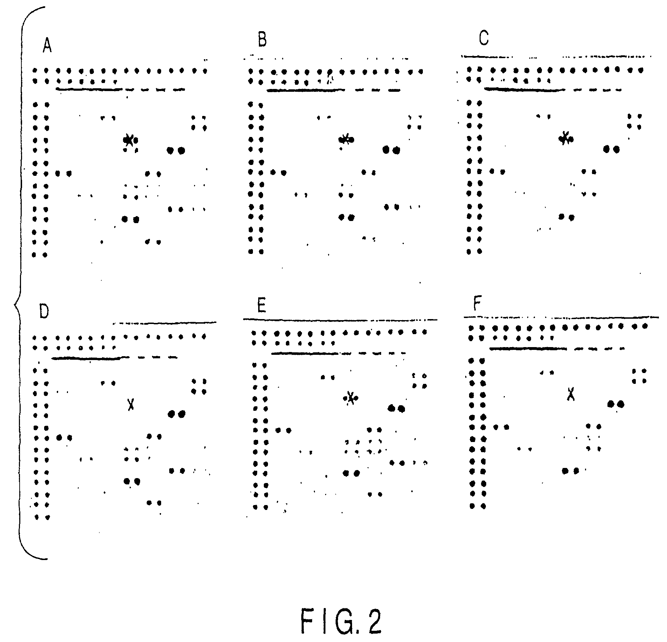

FIG. 2 shows an image of a 1/6 region of a whole DNA micro-array (in this region, black spots are arranged in two rows at the left hand side and in an upper line, for positional confirmation). Genes (7 × 14 = 98), three pairs of reference genes (on solid line) and three pairs of negative control genes (on broken line) are spotted on the right hand side under the upper line.

In FIG. 2, samples A-C, D-F show the results of the samples obtained from schizophrenic patients and non-schizophrenic patients, respectively.

The signal intensity of mRNA of migration-inhibitory factor related protein 8 (MRP-8)(Genbank registration number: X06234, hereinafter referred to as X06234) is indicated by a mark X. The signal intensity of mRNA of Bata-actin X00351 (GenBank registration number:X0035 is seen at a right side of the three pair of reference gene on the solid line). The signal intensity of X06234 is 1.5 times as high as that of X00351 in each of the results A-C obtained from the schizophrenic patients. In contrast, the signal intensity of mRNA of X06234 (indicated by the mark X) is 1.5 times as low as that of the reference gene pair X00351 in each of the non-schizophrenic persons D-F.

When the DNA micro-array is employed as a method of measuring gene expression, it is possible to diagnose schizophrenia by comparing the signal intensity of the mRNA of X00351 (serving as an internal reference) with that of mRNA of an index gene (X06234) of only patient's sample. It is therefore demonstrated that schizophrenia can be diagnosed by using an expression amount of a single gene derived from a patient.

The DNA micro array is advantageous since it makes it possible to measure a plurality of specimens for a short time.

According to the method of the present invention, it is possible to objectively measure whether or nor the subject suffers from schizophrenia. The method of the present invention is excellent in accuracy compared to a conventional subjective diagnostic method.

## Claims

1. A method of diagnosing whether or not said subject suffers from schizophrenia, **characterized by** comprising the steps of:
taking a sample containing, for example, nucleic acid and/or protein from the subject;
quantifying nucleic acid and/or protein corresponding to at least one gene selected from the group consisting of genes listed below, fragments thereof, and complementary nucleic acid thereof:
CCAAT-binding transcription factor subunit B (M59079);
Transcription regulating interferon stimulating gene factor 3 γ subunit (M87503);
DNA topoisomerase 1 (JO3250);
Migration inhibitory factor related protein 8 (X06234);
Growth arrest & DNA-damage inducible protein (M60974);
MacMARCKS(X70326);
ERBB-3 receptor protein-tyrosine kinase precursor (M29366, M34309);
Proto-oncogene c-jun (JO4111);
Phospholipase A2 (M86400);
Erythrocyte urea transporter (U35735);
T-lymphocyte maturation-associated protein MAL(M15800);
Calcium/calmodulin-dependent protein kinase type IV catalytic subunit(L24959);
Interleukin-10 precursor (M57627);
Vascular endothelial growth factor precursor (M32977, M27281);
Defender against cell death 1 (D15057);
Zinc-finger DNA-binding protein (D45132);
Bc12 homologous antagonist/killer (U23765; U16811; X84213);
3'5'-cAMP phosphodiesterase HPDE4A6 (U18087);
Xeroderma pigmentosum group D complementing protein (X52221);
Endothelin receptor type A (L06622),
Epithelial discoidin domain receptor 1 precursor(X74979);
Tyk2 non-receptor protein tyrosine kinase (X54637);
Ets-associated protein tel (U11732);
Platelet-derived growth factor A subunit precursor (X06374);
FAN protein (X96586);
Protein-tyrosine phosphatase γ precursor (L09247);
EB1 protein (U24166);
Ras related protein RAP-1A (M22995);
Myelin-associated oligodendrocytic basic protein (D28113);
Myelin basic protein (M13577);
Brain-derived neurotrophic factor receptor (U12140);
Gamma-aminobutyric acid (GABA) receptor β-1 subunit precursor (X14767);
23k-Da highly basic protein (X56932);
phosphatidylinositol-4-phosphate-5-kinase type III (S78798+U14957);
Recoverin (S43855),
HLA class histocompatibility antigen C-4 α subunit(M11886);
P21-activated kinase α (U24152);
Brain-specific tubulin α1 subunit (K00558);
Ras related protein RAB-11B (X79780);
Bone morphogenetic protein 3 (M22491);
Apoptosis regulator bcl 2 (M14745);
Xenoderma pigmentosum group B complementing protein (M31899);
Acidic fibroblast growth factor (X65778+X51943+M13361);
Neural cell adhesion molecule phosphatidylinositol-linked isoform precursor (X16841; S71824);
Bc12 and p53 linked protein Bbp (U58334);
Induced myeloid leukemia cell differentiation protein MCL-1(L08246);
CD59 glycoprotein precursor (M334671);
Neurotrophin-4 (M86528+S41522+S41540+S41541);
Bone morphogenetic protein 2A (M22489);
ERBB2 receptor protein-tyrosine kinase (M95667+11730);
DAXX (AF015956); and
Apoptosis regulator bax (L22474)
diagnosing whether or not the subject suffers from schizophrenia by using a quantitative value of said at least one nucleic acid.

2. A method for determining whether or not an animal subject is suitable as an animal model for schizophrenia, **characterized by** comprising the steps of:
diagnosing whether or not the animal subject suffers from schizophrenia by the method according to claim 1; and
determining that the animal subject is useful as an animal model if the animal subject suffers from schizophrenia.

3. A method of screening a possible substance as an anti-schizophrenic drug from predetermined test substances, **characterized by** comprising the steps of:
giving the predetermined test substances to an animal model for schizophrenia;
diagnosing whether or not the schizophrenic animal model is recovered from schizophrenia or improved in schizophrenic condition by the method according to claim 1; and
determining that the predetermined test substances are a possible anti-schizophrenic drug if the schizophrenic animal model is recovered from schizophrenia or improved in schizophrenic condition.
